# EUROPEAN PATENT APPLICATION

(11) **EP 3 842 068 A1**
(43) Date of publication of application: **30.06.2021**
(21) Application number: 20213823.6
(22) Date of filing: 05.07.2013
(51) Int. Cl.: A61K 39/12

(54) **NOVEL PRIME-BOOSTING REGIMENS INVOLVING IMMUNOGENIC POLYPEPTIDES ENCODED BY POLYNUCLEOTIDES**

(30) Priority: 05.07.2012 WO PCT/EP2012/063196
(62) Divisional of application: 13734111.1
(71) Applicant: GlaxoSmithKline Biologicals S.A., 1330 Rixensart (BE)
(72) Inventor: NICOSIA, Alfredo, 80123 Naples (IT); CORTESE, Ricardo, 4015 Basel (CH); VITELLI, Alessandra, 00152 Rome (IT)
(74) Representative: Thornley, Rachel Mary

(57) **Abstract**

The present invention relates to administration regimens which are particularly suited for vaccine composition comprising polynucleotides which encode immunogenic polypeptides. Said administration regimens involve the repeated administration of a vaccine composition and enhance the immune response against the immunogenic polypeptide.

## Description

The present invention relates to administration regimens which are particularly suited for vaccine composition comprising polynucleotides which encode immunogenic polypeptides. Said administration regimens involve the repeated administration of a vaccine composition and enhance the immune response against the immunogenic polypeptide.

### Background of the invention

Infectious diseases are still a major threat to mankind. One way for preventing or treating infectious diseases is the artificial induction of an immune response by vaccination which is the administration of antigenic material to an individual such that an adaptive immune response against the respective antigen is developed. The antigenic material may be pathogens (e.g. microorganisms or viruses) which are structurally intact but inactivated (i.e. non-infective) or which are attenuated (i.e. with reduced infectivity), or purified components of the pathogen that have been found to be highly immunogenic. Another approach for inducing an immune response against a pathogen is the provision of expression systems comprising one or more vector encoding immunogenic proteins or peptides of the pathogen. Such vector may be in the form of naked plasmid DNA, or the immunogenic proteins or peptides are delivered by using viral vectors, for example on the basis of modified vaccinia viruses (e.g. Modified Vaccinia Ankara; MVA) or adenoviral vectors. Such expression systems have the advantage of comprising well characterized components having a low sensitivity against environmental conditions.

It is a particular aim when developing vector based expression systems that the application of these expression systems to a patient elicits an immune response which is protective against the infection by the respective pathogen. However, although inducing an immunogenic response against the pathogen, some expression systems are not able to elicit an immune response which is strong enough to fully protect against infections by the pathogen. Accordingly, there is still a need for improved expressions systems which are capable of inducing a protective immune response against a pathogen as well as for novel administration regimens of known expression systems which elicit enhanced immune responses.

### Summary of the Invention

In a first aspect, the present invention relates to a vaccine combination comprising:
(a) a priming composition comprising a first vector comprising a nucleic acid construct encoding at least one immunogenic polypeptide and
(b) at least one boosting composition comprising a second vector comprising a nucleic acid construct encoding at least one immunogenic polypeptide,
wherein at least one epitope of the immunogenic polypeptide encoded by the nucleic acid construct comprised in the first vector is immunologically identical to at least one epitope of the immunogenic polypeptide encoded by the nucleic acid construct comprised in the second vector, for use in a prime-boost vaccination regimen, wherein
(i) the priming composition is administered intranasally and at least one boosting composition is subsequently administered intramuscularly;
(ii) the priming composition is administered intranasally and at least one boosting composition is subsequently administered intranasally.
(ii) the priming composition is administered intramuscularly and at least one boosting composition is subsequently administered intramuscularly; or
(iv) the priming composition is administered intramuscularly and at least one boosting composition is subsequently administered intranasally.

In another aspect, the present invention relates to a vaccine combination comprising:
(a) a priming composition comprising a vector comprising a nucleic acid construct encoding at least one immunogenic polypeptide and
(b) at least one boosting composition comprising at least one immunogenic polypeptide, wherein at least one epitope of the immunogenic polypeptide encoded by the nucleic acid construct comprised in the priming composition is immunologically identical to at least one epitope of the immunogenic polypeptide comprised in the boosting composition, for use in a prime-boost vaccination regimen, wherein the priming composition is administered intramuscular and at least one boosting composition is subsequently administered

### Detailed Description of the Invention

Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. All definitions provided herein in the context of one aspect of the invention also apply to the other aspects of the invention.

In the study underlying the present invention it has been found that specific administration regimens significantly increase the immunity conferred by the vaccine compositions comprising vectors which comprise polynucleotides encoding immunogenic peptides.

Thus, in a first aspect the present invention relates to a vaccine combination comprising:
(a) a priming composition comprising a first vector comprising a nucleic acid construct encoding at least one immunogenic polypeptide and
(b) at least one boosting composition comprising a second vector comprising a nucleic acid construct encoding at least one immunogenic polypeptide,
wherein at least one epitope of the immunogenic polypeptide encoded by the nucleic acid construct comprised in the first vector is immunologically identical to at least one epitope of the immunogenic polypeptide encoded by the nucleic acid construct comprised in the second vector, for use in a prime-boost vaccination regimen, wherein:
(i) the priming composition is administered intranasally and at least one boosting composition is subsequently administered intramuscularly;
(ii) the priming composition is administered intranasally and at least one boosting composition is subsequently administered intranasally.
(ii) the priming composition is administered intramuscularly and at least one boosting composition is subsequently administered intramuscularly; or
(iv) the priming composition is administered intramuscularly and at least one boosting composition is subsequently administered intranasally.
In some instances, the preferred prime-boost vaccination regimen is (i) as it provides a particular effective protective immunity, e.g., by eliciting a strong immune response in the nose and upper respiratory tract.

### Vectors

As used herein, the term "vector" refers to at least one polynucleotide or to a mixture of at least one polynucleotide and at least one protein which is capable of introducing the polynucleotide comprised therein into a cell. At least one polynucleotide comprised by the vector consists of or comprises at least one nucleic acid construct encoding at least one immunogenic protein. In addition to the polynucleotide consisting of or comprising the nucleic acid construct of the present invention additional polynucleotides and/or polypeptides may be introduced into the cell. The addition of additional polynucleotides and/or polypeptides is especially desirable if said additional polynucleotides and/or polypeptides are required to introduce the nucleic acid construct of the present invention into the cell or if the introduction of additional polynucleotides and/or polypeptides increases the expression of the immunogenic polypeptide encoded by the nucleic acid construct of the present invention.

In the context of the present invention it is preferred that the immunogenic polypeptide or polypeptides encoded by the introduced nucleic acid construct are expressed within the cell upon introduction of the vector or vectors. Examples of suitable vectors include but are not limited to plasmids, cosmids, phages, viruses or artificial chromosomes.

In certain preferred embodiments, the first and second vector comprising the nucleic acid constructs of the present invention are selected from the group consisting of plasmids, cosmids, phages, viruses, and artificial chromosomes. More preferably, a vector suitable for practicing the present invention is a phage vector, preferably lambda phage and filamentous phage vectors, or a viral vector.

Suitable viral vectors are based on naturally occurring vectors, which are modified to be replication incompetent also referred to as non-replicating. Non-replicating viruses require the provision of proteins in trans for replication. Typically those proteins are stably or transiently expressed in a viral producer cell line, thereby allowing replication of the virus. The viral vectors are, thus, preferably infectious and non-replicating. The skilled person is aware of how to render various viruses replication incompetent.

In a preferred embodiment of the present invention the vector is selected from the group consisting of adenovirus vectors, adeno-associated virus (AA V) vectors (e.g., AA V type 5 and type 2), alphavirus vectors (e.g., Venezuelan equine encephalitis virus (VEE), sindbis virus (SIN), semliki forest virus (SFV), and VEE-SIN chimeras), herpes virus vectors (e.g. vectors
derived from cytomegaloviruses, like rhesus cytomegalovirus (RhCMV) (14)), arena virus vectors (e.g. lymphocytic choriomeningitis virus (LCMV) vectors (IS)), measles virus vectors, pox virus vectors (e.g., vaccinia virus, modified vaccinia virus Ankara (MVA), NYVAC (derived from the Copenhagen strain of vaccinia), and avipox vectors: canarypox (ALVAC) and fowlpox (FPV) vectors), vesicular stomatitis virus vectors, retrovirus, lentivirus, viral like particles, and bacterial spores.

In particular embodiments, the preferred vectors are adenoviral vectors, in particular adenoviral vectors derived from human or non-human great apes and poxyviral vectors, preferably MVA. Preferred great apes from which the adenoviruses are derived are Chimpanzee (Pan), Gorilla (Gorilla) and orangutans (Pongo), preferably Bonobo *(Pan paniscus)* and common Chimpanzee *(Pan troglodytes).* Typically, naturally occurring non-human great ape adenoviruses are isolated from stool samples of the respective great ape. The most preferred vectors are nonreplicating adenoviral vectors based on hAdS, hAd 1 1, hAd26, hAd3S, hAd49 , ChAd3, ChAd4, ChAdS, ChAd6, ChAd7, ChAd8, ChAd9, ChAdlO, ChAd 1 1, ChAd16, ChAd17, ChAd19, ChAd20, ChAd22, ChAd24, ChAd26, ChAd30, ChAd31, ChAd37, ChAd38, ChAd44, ChAd55, ChAd63, ChAd 73, ChAd82, ChAd83, ChAd146, ChAd147, PanAd1, PanAd2, and PanAd3 vectors or replication-competent Ad4 and Ad7 vectors. The human adenoviruses hAd4, hAdS, hAd7, hAd11, hAd26, hAd35 and hAd49 are well known in the art. Vectors based on naturally occurring ChAd3, ChAd4, ChAd5, ChAd6, ChAd7, ChAd8, ChAd9, ChAd10, ChAd11, ChAd16, ChAd17, ChAd19, ChAd20, ChAd22, ChAd24, ChAd26, ChAd30, ChAd31, ChAd37, ChAd38, ChAd44 , ChAd63 and ChAd82 are described in detail in WO2005/071093. Vectors based on naturally occurring PanAd1, PanAd2, PanAd3, ChAd55, ChAd73, ChAd83, ChAd146, and ChAd147 are described in detail in WO 2010/086189.

The term "non-replicating adenovirus" refers to an adenovirus that has been rendered to be incapable of replication because it has been engineered to comprise at least a functional deletion, or a complete removal of, a gene product that is essential for viral replication, such as one or more of the adenoviral genes selected from E1, E2, E3 and E4.

Preferrably the first vector used is an adenoviral vector, more preferably non-human great ape, e.g. a chimpanzee or bonobo, derived adenoviral vector, in particular a.non-replicating adenoviral vector based on ChAd3, ChAd4, ChAdS, ChAd6, ChAd7, ChAd8, ChAd9, ChAd10, ChAd11, ChAd16, ChAd17, ChAd19, ChAd20, ChAd22, ChAd24, ChAd26, ChAd30, ChAd31, ChAd37, ChAd38, ChAd44, ChAd55, ChAd63, ChAd73, ChAd82, ChAd83, ChAd146, ChAd147, PanAd1, PanAd2, and PanAd3 or replication-competent vector based on hAd4 and hAd7. The most preferred vector is based on PanAd3.

Preferably, the second vector is a poxyviral vector, particularly MVA or an adenoviral vector, preferably a non-human great ape derived adenoviral vector. Preferred non-replicating adenoviral vectors are based on ChAd3, ChAd4, ChAd5, ChAd6, ChAd7, ChAd8, ChAd9, ChAd10, ChAd11, ChAd16, ChAd17, ChAd19, ChAd20, ChAd22, ChAd24, ChAd26, ChAd30, ChAd31, ChAd37, ChAd38, ChAd44, ChAd55, ChAd63, ChAd73, ChAd82, ChAd83, ChAd146, ChAd147, PanAd1, PanAd2, and PanAd3 vectors or replication-competent Ad4 and Ad7 vector.

If the first and the second vector are adenoviral vectors, it is sometimes preferred to use immunologically different adenoviral vectors as first and second vectors. If both vectors are immunologically identical, there can be a potential risk that antibodies generated against the first vector during priming of the immune response impair the transduction of the patient with the second vector used for boosting the immune response. Adenoviruses and, thus, adenoviral vectors typically comprise three envelope proteins, i.e. hexon, penton and fibre. The immunological response of a host against a given adenovirus is primarily determined by the hexon protein. Thus, two adenoviruses are considered to be immunologically different within the meaning of the present invention, if the hexon proteins of the two adenoviruses differ at least in one epitope. The T-cell and B-cell epitopes of hexon have been mapped.

In one particular preferred embodiment of the present invention, the first vector is an adenoviral vector, in particular PanAd3, and the second vector is a poxyviral vector, in particular MVA, or an adenoviral vector.

In one preferred embodiment of the present invention, the first vector is PanAd3 and the second vector is MV A. A description of MV A can be found in Mayr A, Stickl H, Muller HK, Danner K, Singer H. "The smallpox vaccination strain MVA: marker, genetic structure, experience gained with the parenteral vaccination and behavior in organisms with a debilitated defence mechanism." Zentralbl Bakteriol B. 1978 Dec; 167(5-6):375-90 and in Mayr, A., Hochstein-Mintzel, V. & Stickl, H. (1975). "Abstammung, Eigenschaften und Verwendung des attenuierten Vaccinia-Stammes MVA." Infection 3, 6-14.

The terms "polynucleotide" and "nucleic acid" are used interchangeably throughout this application. Polynucleotides are understood as a polymeric macromolecules made from nucleotide monomers. Nucleotide monomers are composed of a nucleobase, a five-carbon sugar (such as but not limited to ribose or 2'-deoxyribose), and one to three phosphate groups. Typically, a polynucleotide is formed through phosphodiester bonds between the individual nucleotide monomers. In the context of the present invention preferred nucleic acid molecules include but are not limited to ribonucleic acid (RNA) and deoxyribonucleic acid (DNA).

Moreover, the term "polynucleotide" also includes artificial analogs of DNA or RNA, such as peptide nucleic acid (PNA).

Additional suitable vectors are described in detail in PCT/EP20 111074307. The disclosure of this application is herewith incorporated by reference with respect to its disclosure relating to the expression systems disclosed therein.

### Polypeptides

The terms "protein", "polypeptide" and "peptide" are used interchangeably herein and refer to any peptide-linked chain of amino acids, regardless of length co-translational or post-translational modification.

The term "post-translational" used herein refers to events that occur after the translation of a nucleotide triplet into an amino acid and the formation of a peptide bond to the preceeding amino acid in the sequence. Such post-translational events may occur after the entire polypeptide was formed or already during the translation process on those parts of the polypeptide that have already been translated. Post-translational events typically alter or modify the chemical or structural properties of the resultant polypeptide. Examples of post-translational events include but are not limited to events such as glycosylation or phosphorylation of amino acids, or cleavage of the peptide chain, e.g. by an endopeptidase.

The term "co-translational" used herein refers to events that occur during the translation process of a nucleotide triplet into an amino acid chain. Those events typically alter or modify the chemical or structural properties of the resultant amino acid chain. Examples of cotranslational events include but are not limited to events that may stop the translation process entirely or interrupt the peptide bond formation resulting in two discreet translation products.

As used herein, the terms "polyprotein" or "artificial polyprotein" refer to an amino acid chain that comprises, or essentially consists of or consists of two amino acid chains that are not naturally connected to each other. The polyprotein may comprise one or more further amino acid chains. Each amino acid chain can be a complete protein, i.e. spanning an entire ORF, or a fragment, domain or epitope thereof. The individual parts of a polyprotein may either be permanently or temporarily connected to each other. Parts of a polyprotein that are permanently connected are translated from a single ORF and are not later separated co- or post-translationally. Parts of polyproteins that are connected temporarily may also derive from a single ORF but are divided co-translationally due to separation during the translation process or post-translationally due to cleavage of the peptide chain, e.g. by an endopeptidase. Additionally or alternatively, parts of a polyprotein may also be derived from two different ORF and are connected post-translationally, for instance through covalent bonds.

Proteins or polyproteins usable in the present invention (including protein derivatives, protein variants, protein fragments, protein segments, protein epitopes and protein domains) can be further modified by chemical modification. Hence, such a chemically modified polypeptide may comprise chemical groups other than the residues found in the 20 naturally occurring amino acids. Examples of such other chemical groups include without limitation glycosylated amino acids and phosphorylated amino acids. Chemical modifications of a polypeptide may provide advantageous properties as compared to the parent polypeptide, e.g. one or more of enhanced stability, increased biological half-life, or increased water solubility. Chemical modifications applicable to the variants usable in the present invention include without limitation: PEGylation, glycosylation of non-glycosylated parent polypeptides, or the modification of the glycosylation pattern present in the parent polypeptide. Such chemical modifications applicable to the variants usable in the present invention may occur co-or post-translational.

An "immunogenic polypeptide" as referred to in the present application is a polypeptide as defined above which contains at least one epitope. An "epitope", also known as antigenic determinant, is that part of a polypeptide which is recognized by the immune system. Preferably, this recognition is mediated by the binding of antibodies, B cells, or T cells to the epitope in question. In this context, the term "binding" preferably relates to a specific binding. Preferably, the specific binding of antibodies to an epitope is mediated by the Fab (fragment, antigen binding) region of the antibody, specific binding of a B-cell is mediated by the Fab region of the antibody comprised by the B-cell receptor and specific binding of a T-cell is mediated by the variable (V) region of the T-cell receptor.

An immunogenic polypeptide according to the present invention is, preferably, derived from a pathogen selected from the group consisting of viruses, bacteria and protozoa. In particular embodiments, it is derived from a virus and, in one particularly favorable embodiment, it is derived from respiratory syncytial virus (RSV). However, in an alternative embodiment of the present invention the immunogenic polypeptide is a polypeptide or fragment of a polypeptide expressed by a cancer.

Preferred immunogenic polypeptides induce a B-cell response or a T-cell response or a B cell response and a T-cell response.

Epitopes usually consist of chemically active surface groupings of molecules such as amino acids or sugar side chains and usually have specific three-dimensional structural characteristics, as well as specific charge characteristics. The term "epitope" refers to conformational as well as non-conformational epitopes. Conformational and non-conformational epitopes are distinguished in that the binding to the former but not the latter is lost in the presence of denaturing solvents.

Two or more immunogenic polypeptides are "immunologically identical" if they are recognized by the same antibody, T-cell or B-cell. The recognition of two or more immunogenic polypeptides by the same antibody, T-cell or B-cell is also known as "cross reactivity" of said antibody, T-cell or B-cell. The recognition of two or more immunologically identical polypeptides by the same antibody, T-cell or B-cell is due to the presence of identical or similar epitopes in all polypeptides. Similar epitopes share enough structural and/or charge characteristics to be bound by the Fab region of the same antibody or B-cell receptor or by the V region of the same T-cell receptor. The binding characteristics of an antibody, T-cell receptor or B-cell receptor are, typically, defined by the binding affinity of the receptor to the epitope in question. Two immunogenic polypeptides are "immunologically identical" as understood by the present application if the affinity constant of polypeptide with the lower affinity constant is at least 30 %, at least 40 %, at least 50 %, at least 60 %, at least 70 %, at least 80 %, at least 90 %, at least 95 % or at least 98 % of the affinity constant of the polypeptide with the higher affinity constant. Methods for determining the binding affinity of a polypeptide to a receptor such as equilibrium dialysis or enzyme linked immunosorbent assay (ELISA) are well known in the art.

Preferably, two or more "immunologicaly identical" polypeptides comprise at least one identical epitope. The strongest vaccination effects can usually be obtained, if the immunogenic polypeptides comprise identical epitopes or if they have an identical amino acid sequence.

As used herein, a polypeptide whose amino acid sequence is "substantially identical" to the amino acid sequence of another polypeptide is a polypeptide variant which differs in comparison to the other polypeptide (or segment, epitope, or domain) by one or more changes in the amino acid sequence. The polypeptide from which a protein variant is derived is also known as the parent polypeptide. Typically, a variant is constructed artificially, favorably by gene-technological means. Typically, the parent polypeptide is a wild-type protein or wild-type protein domain. **In** the context of the present invention, a parent polypeptide (or parent segment) can also be the consensus sequence of two or more wild-type polypeptides (or wild-type segments). Further, the variants usable in the present invention may also be derived from homologs, orthologs, or paralogs of the parent polypeptide or from an artificially constructed variant, provided that the variant exhibits at least one biological activity of the parent polypeptide. Preferably, the at least one biological activity of the parent polypeptide shared by the variant is (or includes) the presence of at least one epitope which renders both polypeptides "immunologically identical" as defined above.

The changes in the amino acid sequence may be ammo acid exchanges, insertions, deletions, N-terminal truncations, or C-terminal truncations, or any combination of these changes, which may occur at one or several sites. In certain favorable embodiments, a variant usable in the present invention exhibits a total number of up to 200 (up to 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, or 200) changes in the amino acid sequence (i.e. exchanges, insertions, deletions, N-terminal truncations, and/or C-terminal truncations). The amino acid exchanges may be conservative and/or non-conservative. In certain favorable embodiments, a variant usable in the present invention differs from the protein or domain from which it is derived by up to 1, 2, 3, 4, 5, 6, 7, 8, 9, 10, 15, 20, 25, 30, 35, 40, 45, 50, 55, 60, 65, 70, 75, 80, 85, 90, 95, or 100 amino acid exchanges, preferably conservative amino acid changes.

Alternatively or additionally, a "variant" as used herein, can be characterized by a certain degree of sequence identity to the parent polypeptide or parent polynucleotide from which it is derived. More precisely, a protein variant which is "substantially identical" to another polypeptide exhibits at least 80% sequence identity to the other polypeptide. A polynucleotide variant in the context of the present invention exhibits at least 80% sequence identity to its parent polynucleotide. Preferably, the sequence identity of protein variants is over a continuous stretch of 20, 30, 40, 45, 50, 60, 70, 80, 90, 100 or more amino acids. Preferably, the sequence identity of polynucleotide variants is over a continuous stretch of 60, 90, 120, 135, 150, 180, 210, 240, 270, 300 or more nucleotides.

In a preferred embodiment of the present invention, a polypeptide which is "substantially identical" to its parent polypeptide has at least 80 % sequence identity to said parent polypeptide. More preferably, the said polypeptide is immunologically identical to the parent polypeptide and has at least 80 % sequence identity to the parent polypeptide.

The term "at least 80% sequence identity" is used throughout the specification with regard to polypeptide and polynucleotide sequence comparisons. This expression refers to a sequence identity of at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95 %, at least 96 %, at least 97 %, at least 98 %, or at least 99 % to the respective reference polypeptide or to the respective reference polynucleotide. Preferably, the polypeptide in question and the reference polypeptide exhibit the indicated sequence identity over a continuous stretch of 20, 30, 40, 45, 50, 60, 70, 80, 90, 100 or more amino acids or over the entire length of the reference polypeptide. Preferably, the polynucleotide in question and the reference polynucleotide exhibit the indicated sequence identity over a continuous stretch of60, 90, 120, 135, 150, 180, 210, 240, 270, 300 or more nucleotides or over the entire length of the reference polypeptide.

Variants of a polypeptide may additionally or alternatively comprise deletions of amino acids, which may be N-terminal truncations, C-terminal truncations or internal deletions or any combination of these. Such variants comprising N-terminal truncations, C-terminal truncations and/or internal deletions are referred to as "deletion variant" or "fragments" in the context of the present application. The terms "deletion variant" and "fragment" are used interchangeably herein. A fragment may be naturally occurring (e.g. splice variants) or it may be constructed artificially, for example, by gene-technological means. A fragment (or deletion variant) can have a deletion of up to 1, 2, 3, 4, 5, 6, 7, 8, 9, 10, 15, 20, 25, 30, 35, 40, 45, 50, 55, 60, 65, 70, 75, 80, 85, 90, 95, or 100 amino acids as compared to the parent polypeptide, preferably at the N-terminus, at the N-and C-terminus, or at the C-terminus, or internally. In case where two sequences are compared and the reference sequence is not specified in comparison to which the sequence identity percentage is to be calculated, the sequence identity is to be calculated with reference to the longer of the two sequences to be compared, if not specifically indicated otherwise. If the reference sequence is indicated, the sequence identity is determined on the basis of the full length of the reference sequence indicated by SEQ ID, if not specifically indicated otherwise.

Additionally or alternatively a deletion variant may occur not due to structural deletions of the respective amino acids as described above, but due to these amino acids being inhibited or otherwise not able to fulfill their biological function. Typically, such functional deletion occurs due to the insertions into or exchanges in the amino acid sequence that changes the functional properties of the resultant protein, such as but not limited to alterations in the chemical properties of the resultant protein (i.e. exchange of hydrophobic amino acids to hydrophilic amino acids), alterations in the post-translational modifications of the resultant protein (e.g. post-translational cleavage or glycosylation pattern), or alterations in the secondary or tertiary protein structure. Preferably, a functional deletion as described above, is caused by an insertion or exchange of at least one amino acid which results in the disruption of an epitope of an immunogenic polypeptide.

The similarity of nucleotide and amino acid sequences, i.e. the percentage of sequence identity, can be determined via sequence alignments. Such alignments can be carried out with several art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wust1.edu/) or with the CLUSTAL algorithm (Thompson, 1. D., Higgins, D. G. & Gibson, T. 1. (1994) Nucleic Acids Res. 22, 4673-80) available e.g. on http://www.ebi.ac.uk/Tools/clustalw/ or on http://www.ebi.ac.uk/Tools/clust alw2lindex.html or on http://npsa-pbi1.ibcp.fr/cgi-binlnpsa automat.pl?page=INPSAlnpsa cl ustalw.html. Preferred parameters used are the default parameters as they are set on http://www.ebi.ac.uklTools/clustalw/ or http://www.ebi.ac.uklTools/clustalw2lindex.html. The grade of sequence identity (sequence matching) may be calculated using e.g. BLAST, BLAT or BlastZ (or BlastX). A similar algorithm is incorporated into the BLASTN and BLASTP programs of Altschul et al. (1990) 1. Mol. Biol. 215: 403-410. BLAST polynucleotide searches are performed with the BLASTN program, score = 100, word length = 12, to obtain polynucleotide sequences that are homologous to those nucleic acids which encode F, N, or M2-1. BLAST protein searches are performed with the BLASTP program, score = 50, word length = 3, to obtain amino acid sequences homologous to the F polypeptide, N polypeptide, or M2-1 polypeptide. To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1:154-162) or Markov random fields. When percentages of sequence identity are referred to in the present application, these percentages are calculated in relation to the full length of the longer sequence, if not specifically indicated otherwise.

The polynucleotides of the invention encodes proteins, peptides or variants thereof which comprise amino acids which are designated following the standard one-or three-letter code according to WIPO standard ST.25 unless otherwise indicated. If not indicated otherwise, the one-or three letter code is directed at the naturally occurring L-amino acids and the amino acid sequence is indicated in the direction from the N-terminus to the C-terminus of the respective protein, peptide or variant thereof.

As used herein, the term "consensus" refers to an amino acid or nucleotide sequence that represents the results of a multiple sequence alignment, wherein related sequences are compared to each other. Such a consensus sequence is composed of the amino acids or nucleotides most commonly observed at each position. In the context of the present invention it is preferred that the sequences used in the sequence alignment to obtain the consensus sequence are sequences of different viral subtypes/serotypes strains isolated in various different disease outbreaks worldwide. Each individual sequence used in the sequence alignment is referred to as the sequence of a particular virus "isolate". In case that for a given position no "consensus nucleotide" or "consensus amino acid" can be determined, e.g. because only two isolates were compared, it is preferred that the amino acid of each one of the isolates is used.

The phrase "induction of a T cell response" refers to the generation or the restimulation of pathogen specific, preferably virus specific, CD4+ or CD8+ T cells. In one embodiment of the
present invention, the priming composition and/or the boosting composition can induce or re-stimulate a T cell mediated adaptive response directed to the MHC class I or class II epitopes present in the polypeptide or polypeptides expressed by the nucleic acid construct. Such T cell response can be measured by art known methods, for example, by ex-vivo re-stimulation of T cells with synthetic peptides spanning the entire polypeptide and analysis of proliferation or Interferon-gamma production.

The phrase "induction of a B cell response" refers to the generation or the restimulation of pathogen specific, for example, virus specific, B cells producing immunoglobulins of class IgG or IgA. In one embodiment of the present invention, the priming composition and/or the boosting composition can induce or re-stimulate B cells producing antibodies specific for pathogenic, e.g. viral, antigens, expressed by the nucleic acid construct. Such B cell response can be measured by ELISA with the synthetic antigen of serum or mucosal immunoglobulin. Alternatively the induced antibody titer can be measured by virus neutralization assays.

The phrase "induction of an anti-pathogenic B cell response" refers to the generation or the re-stimulation of pathogen specific, such as virus specific, B cells producing immunoglobulins of class IgG or IgA which inactivate, eliminate, blocks and/or neutralize the respective pathogen such that the disease caused by the pathogen does not break out and/or the symptoms are alleviated. This is also called a "protective immune response" against the pathogen. In a preferred embodiment of the present invention, the priming and/or boosting composition of the invention can induce or re-stimulate B cells producing antibodies specific for pathogenic, e.g. viral, antigens expressed by the nucleic acid construct. Such B cell response can be measured by ELISA with the synthetic antigen of serum or mucosal immunoglobulin. Alternatively the induced antibody titer can be measured by virus neutralization assays.

The phrase "enhancing an immune response" refers to the strengthening or intensification of the humoral and/or cellular immune response against an immunogen, preferably pathogens, such as viruses. The enhancement of the immune response can be measured by comparing the immune response elicited by an expression system of the invention with the immune response of an expression system expressing the same antigen/immunogen alone by using tests described herein and/or tests well known in the present technical field.

Suitable immunogenic polypeptides are described in detail in PCT/EP2011/074307. The disclosure of this application is herewith incorporated by reference with respect to its disclosure relating to the immunogenic polypeptides disclosed therein.

In certain preferred embodiments, the immunogenic polypeptides are described below using the following abbreviations: "F" or "FO" are used interchangeably herein and refer to the Fusion protein of paramyxoviruses, preferably of RSV; "G" refers to the Glycoprotein of paramyxoviruses, preferably of pneumovirinae, more preferably of RSV; H" refers to the Hemagglutinin Protein of paramyxoviruses, preferably of morbilliviruses; "HN" refers to the Hemagglutinin-Neuraminidase Protein of paramyxoviruses, particularly of Respirovirus, Avulavirus and Rubulavirus; "N" refers to the Nucleocapsid protein of paramyxoviruses, preferably of RSV; "M" refers to the glycosylated Matrix protein of paramyxoviruses, preferably of RSV; with respect to paramyxoviruses, the abbreviation "M2" or "M2-1" refers to the non-glycosylated Matrix protein of paramyxoviruses, preferably of RSV; "P" refers to the Phosphoprotein of paramyxoviruses, preferably of RSV; with respect to paramyxoviruses, the abbreviation "NS 1" and "NS2" refer to the non-structural proteins 1 and 2 of paramyxoviruses, preferably of RSV; "L" refers to the catalytic subunit of the polymerase of paramyxoviruses, preferably of RSV; "HA" refers to the hemagglutinin of orthomyxovirus, preferably influenzaviruses, more preferably of influenza A virus; "HAO" refers to the precursor protein of hemagglutinin subunits HAl and HA2 of orthomyxovirus, preferably influenzaviruses, more preferably of influenza A virus; "Hlp" refers to the modified hemagglutinin of orthomyxovirus, preferably influenzaviruses, more preferably of influenza A virus; "NA" refers to the neuraminidase of orthomyxovirus, preferably influenzaviruses, more preferably of influenza A virus; "NP" refers to the nucleoprotein of orthomyxoviruses, preferably influenzaviruses, more preferably of influenza A virus; "MI" refers to the matrixprotein 1 of orthomyxoviruses, preferably influenzaviruses, more preferably of influenza A virus; with respect to orthomyxoviruses, the abbreviation "M2" refers to the Matrix protein M2 of orthomyxoviruses, preferably influenzaviruses, more preferably of influenza A virus; with respect to orthomyxovirus, the abbreviation "NS 1" refers to the non-structural protein 1 of orthomyxoviruses, preferably influenzaviruses, more preferably of influenza A Virus; "NS2/NEP" refers to the non-structural protein 2 (also referred to as NEP, nuclear export protein) of orthomyxoviruses, preferably influenzaviruses, more preferably influenza A virus; "PA" refers to a polymerase subunit protein of orthomyxoviruses, preferably influenzaviruses, more preferably influenza A virusM "PB 1" refers to a polymerase subunit protein of orthomyxoviruses, preferably influenzaviruses, more preferably influenza A virus; "PB2" refers to a polymerase subunit protein of orthomyxoviruses, preferably influenzaviruses, more preferably influenza A virus; "PB1-F2" or "PB1F2" refers to a protein encoded by an alternate reading frame in the PB1 Gene segment of orthomyxoviruses, preferably influenzaviruses, more preferably influenza A virus.

In other preferred embodiments, the immunogenic polypeptides are tumor-specific proteins or pathogen specific proteins. In certain embodiments, the pathogens are viruses, in particular paramyxovirus or variants thereof, preferably selected from the subfamily of Pneumovirinae, Paramyxovirinae, Fer-de-Lance-Virus, Nariva-Virus, Salem-Virus, Tupaia-Paramyxovirus, Beilong-Virus, 1-Virus, Menangle-Virus, Mossmann-Virus, and Murayama-Virus. In even more preferred embodiments, the Pneumovirinae is selected from the group consisting of Pneumovirus, preferably human respiratory syncytial virus (RSV), murine pneumonia virus, bovine RSV, ovine RSV, caprine RSV, turkey rinotracheitis virus, and Metapneumovirus, preferably human metapneumovirus (hMPV) and avian metapneumovirus. In even more preferred embodiments, the Paramyxovirinae is selected from the group consisting of Respirovirus, preferably human parainfluenza virus 1 and 3, and Rubulavirus, preferably human parainfluenza virus 2 and 4; bacteria, or protozoa, preferably *Entomoeba histolytica, Trichomonas tenas, Trichomonas hominis, Trichomonas vagina lis* , *Trypanosoma gambiense, Trypanosoma rhodesiense, Trypanosoma cruzi, Leishmania donovani, Leishmania tropica, Leishmania braziliensis, Pneumocystis pneumonia, Toxoplasma gondii, Theileria lawrenci, Theileria parva, Plasmodium vivax, Plasmodium falciparum,* and *Plasmodium malaria.*

### Nucleic acid constructs

The term "nucleic acid construct" refers to a polynucleotide which encodes at least one immunogenic polypeptide. Preferably, said polynucleotide additionally comprises elements which direct transcription and translation of the at least one polypeptide encoded by the nucleic acid construct. Such elements include promoter and enhancer elements to direct transcription of mRNA in a cell-free or a cell-based based system, preferably a cell-based system. In another embodiment, wherein the nucleic acid construct is provided as translatable RNA, it is envisioned that the nucleic acid construct comprises those elements that are necessary for translation and/or stabilization of RNAs encoding the at least one immunogenic polypeptide, e.g. polyA-tail, IRES, cap structures etc.

As outlined above, it is preferred that the vector of the present invention is a viral vector and, thus, the nucleic acid construct is preferably comprised by a larger polynucleotide which additionally includes nucleic acid sequences which are required for the replication of the viral vector and/or regulatory elements directing expression of the immunogenic polypeptide.

In one embodiment of the present invention, the nucleic acid construct encodes a single immunogenic polypeptide.

In a specific preferred embodiment of the present invention, the nucleic acid construct encodes at least two immunogenic polypeptides.

Suitable nucleic acid constructs encoding immunogenic polypeptides are described in detail in PCT/EP2011/074307. The disclosure of this application is herewith incorporated by reference with respect to its disclosure relating to the immunogenic polypeptides disclosed therein.

It has been surprisingly found in the study underlying PCT/EP201/074307 that the addition of an immunogenic polypeptide which induces a T-cell response to an immunogenic polypeptide which induces a B-cell response enhances the B-cell response against the latter polypeptide. Methods for determining the strength of a B-cell response against an antigen described above. The titer of antibodies specific for the antigen in question can be determined at least 2 weeks, at least 4 weeks, at least 8 weeks, at least 4 months, at least 8 months or at least 1 year after immunization with a combination of at least one immunogenic polypeptide inducing a B-cell response and at least one immunogenic polypeptide inducing a T-cell response. Preferably, the titer of antibodies specific for the immunogenic polypeptide inducing a B-cell response is increased by the combination by at least 10 %, at least 20 %, at least 30 %, at least 50 %, at least 75 %, at least 100 %, at least 150 % or at least 200 % as compared to immunization with the at least one immunogenic polypeptide inducing a B-cell response alone.

Therefore, in a preferred embodiment of the present invention, the nucleic acid construct encodes at least one immunogenic polypeptide inducing a B-cell response and at least one immunogenic polypeptide inducing a T-cell response.

The immunogenic polypeptide which induces a B-cell response is, preferably, a structural protein comprised by a virus or a fragment or variant thereof. For example, in the case of a enveloped viruses, the structural viral protein can favorably be selected from the group consisting of fusion protein (F) and attachment glycoproteins G, H, and HN.

The attachment glycoproteins are found in all enveloped viruses and mediate the initial interaction between the viral envelope and the plasma membrane of the host cell via their binding to carbohydrate moieties or cell adhesion domains of proteins or other molecules on the plasma membrane of the host cell. Thereby, attachment glycoproteins bridge the gap between the virus and the membrane of the host cell. Attachment glycoproteins designated as "H" possess hemagglutinin activity and are found in morbilliviruses and henipaviruses, glycoproteins designated as "HN possess hemagglutinin and neuraminidase activities and are found in respiroviruses, rubulaviruses and avulaviruses. Attachment glycoproteins are designated as "G" when they have neither haemagglutination nor neuraminidase activity. G attachment glycoproteins can be found in all members of Pneumovirinae.

Fusion protein "F" is found in all enveloped viruses and mediates the fusion of the viral envelope with the plasma membrane of the host cell. F is a type I glycoprotein that recognizes receptors present on the cell surface of the host cell to which it binds. F consists of a fusion peptide adjacent to which the transmembrane domains are located, followed by two heptad repeat (HR) regions, HRI and HR2, respectively. Upon insertion of the fusion peptide into the plasma membrane of the host cell, the HRI region forms a trimeric coiled coil structure into whose hydrophobic grooves the HR2 regions folds back. Thereby, a hairpin structure is formed that draws the viral lipid bilayer and cellular plasma membrane even closer together and allows for the formation of a fusion pore and consecutively the complete fusion of both lipid bilayers enabling the virus capsid to enter into the cytoplasm of the host cell. All of these features are common in fusion-mediating proteins of enveloped viruses.

In a preferred embodiment of the present invention, F comprises, essentially consists of or consists of an amino acid sequence of F of one RSV isolate or a consensus amino acid sequence of two or more different RSV isolates. In certain preferred embodiments, the amino acid sequence of the F protein is preferably according to SEQ ID NO: 1, SEQ ID NO: 2 or a variant thereof.

The immunogenic polypeptide which induces aT-cell response is, favorably, an internal protein comprised by a virus or a fragment or variant thereof. Said structural viral protein can be selected from the group consisting of nucleoprotein N, Matrix proteins M and M2, Phosphoprotein P, non structural proteins NSI and NS2, and the catalytic subunit of the polymerase (L).

The nucleoprotein N serves several functions which include the encapsidation of the RNA genome into a RNAase-resistant nucleocapsid. N also interacts with the M protein during virus assembly and interacts with the P-L polymerase during transcription and replication of the genome.

The matrix protein M is the most abundant protein in paramyxovirus and is considered to be the central organizer of viral morphology by interacting with the cytoplasmatic tail of the integral membrane proteins and the nucleocapsid. M2 is a second membrane-associated protein that is not glycosylated and is mainly found in pneumovirus.

Phosphoprotein P binds to the N and L proteins and forms part of the RNA polymerase complex in all paramyxoviruses. Large protein L is the catalytic subunit of RNA-dependent RNA polymerase.

The function of non-structural proteins NS 1 and NS2 has not yet been identified; however, there are indications that they are involved in the viral replication cycle.

In certain preferred embodiments, N comprises an amino acid sequence of N, of one RSV isolate or a consensus amino acid sequence of two or more different RSV isolates, e.g., according to SEQ ID NO: 3 and wherein M2 comprises an amino acid sequence of M2 of one RSV isolate or a consensus amino acid sequence of two or more different RSV isolates, e.g., according to SEQ ID NO: 5. In one further preferred embodiment, N comprises the amino acid sequence according to SEQ ID NO: 4 and M2 comprises the amino acid sequence according to SEQ IDNO: 5.

In one preferred embodiment of the present invention the at least two different immunogenic polypeptides are encoded by the same number of open reading frames (ORFs), i.e. each polypeptide is encoded by a separate open reading frame. In this case, it is preferred that each ORF is combined with suitable expression control sequences which allow the expression of said polypeptide.

In another preferred embodiment of the present invention, at least two different immunogenic polypeptides are encoded by a single ORF and linked by a peptide linker. Thus, transcription and translation of the nucleic acid construct result in a single polypeptide having to functional, i.e. immunogenic, domains. The term "different immunogenic polypeptides" refers to immunogenic polypeptides as defined above in this application which are not encoded by a contiguous nucleic acid sequence in the virus or organism they are derived from. In the virus or organism they are derived from, they may be encoded by different ORFs. Alternatively, they may be derived from different domains of a polypeptide encoded by a single ORF by deletion of amino acid sequences which connected said domains in their natural context and the replacement of said connecting amino acid sequences by a peptide linker. The latter embodiment allows the production of a polypeptide shorter than the naturally occurring polypeptide which still contains all epitopes which are necessary for the induction of an immune response. To give an example: a naturally occurring polypeptide comprises two epitopes useful for eliciting an immune response linked by an amino acid sequence of 90 amino acids which is not immunogenic. The replacement of said 90 amino acids by a peptide linker of 10 or 15 amino acids results in a shorter polypeptide which, nevertheless, comprises both important epitopes.

In one particular preferred embodiment of the present invention, at least two different immunogenic polypeptides are encoded by a single ORF and linked by a cleavage site. Thus, transcription and translation of the nucleic acid construct result in a single polypeptide which is cut into different smaller polypeptides co-translationally or post-translationally.

The cleavage referred to above site is, preferably, a self-cleaving or an endopeptidase cleavage site.

The term "open reading frame" (ORF) refers to a sequence of nucleotides, that can be translated into amino acids. Typically, such an ORF contains a start codon, a subsequent region usually having a length which is a multiple of 3 nucleotides, but does not contain a stop codon (TAG, TAA, TGA, UAG, UAA, or UGA) in the given reading frame. Typically, ORFs occur naturally or are constructed artificially, i.e. by gene-technological means. An ORF codes for a protein where the amino acids into which it can be translated form a peptide-linked chain.

A "peptide linker" (or short: "linker") in the context of the present invention refers to an amino acid sequence of between 1 and 100 amino acids. In preferred embodiments, a peptide linker according to the present invention has a minimum length of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids. In further preferred embodiments, a peptide linker according to the present invention has a maximum length of 100, 95, 90, 85, 80, 75, 70, 65, 60, 55, 50, 45, 40, 35, 34, 33, 32, 31, 30, 29, 28, 27, 26, 25, 24, 23, 22, 21, 20, 19, 18, 17, 16, or 15 amino acids or less. It is preferred that peptide linkers provide flexibility among the two amino acid proteins, fragments, segments, epitopes and/or domains that are linked together. Such flexibility is generally increased if the amino acids are small. Thus, preferably the peptide linker of the present invention has an increased content of small amino acids, in particular of glycines, alanines, serines, threonines, leucines and isoleucines. Preferably, more than 20%, 30%, 40%, 50%, 60% or more of the amino acids of the peptide linker are small amino acids. In a preferred embodiment the amino acids of the linker are selected from glycines and serines. In especially preferred embodiments, the above-indicated preferred minimum and maximum lengths of the peptide linker according to the present invention may be combined. One of skill will immediately understand which combinations makes sense mathematically. In certain preferred embodiments, the peptide linker of the present invention is non-immunogenic; and when designed for administration to humans, the peptide linker is typically selected to be non-immunogenic to humans.

The term "cleavage site" as used herein refers to an amino acid sequence where this sequence directs the division, e.g. because it is recognized by a cleaving enzyme, and/or can be divided. Typically, a polypeptide chain is cleaved by hydrolysis of one or more peptide bonds that link the amino acids. Cleavage of peptide bonds may originate from chemical or enzymatic cleavage. Enzymatic cleavage refers to such cleavage being attained by proteolytic enzymes endo-or exo-peptidases or -proteases (e.g. serine-proteases, cysteine-proteases, metalloproteases, threonine proteases, aspartate proteases, glutamic acid proteases). Typically, enzymatic cleavage occurs due to self-cleavage or is effected by an independent proteolytic enzyme. Enzymatic cleavage of a protein or polypeptide can happen either co-or post-translational. Accordingly, the term "endopeptidase cleavage site" used herein, refers to a cleavage cite within the amino acid or nucleotide sequence where this sequence is cleaved or is cleavable by an endopeptidase (e.g. trypsin, pepsin, elastase, thrombin, collagenase, furin, thermolysin, endopeptidase V8, cathepsins). Alternatively or additionally, the polypeptides of the present invention can be cleaved by an autoprotease, i.e. a protease which cleaves peptide
bonds in the same protein molecule which also comprises the protease. Examples of such autoproteases are the NS2 protease from flaviviruses or the VP4 protease of birnaviruses.

Alternatively, the term "cleavage site" refers to an amino acid sequence that prevents the formation of peptide bonds between amino acids. For instance, the bond formation may be prevented due to co-translational self-processing of the polypeptide or polyprotein resulting in two discontinuous translation products being derived from a single translation event of a single open reading frame. Typically, such self-processing is effected by a "ribosomal skip" caused by a pseudo stop-codon sequence that induces the translation complex to move from one codon to the next without forming a peptide bond. Examples of sequences inducing a ribosomal skip include but are not limited to viral 2A peptides or 2A-like peptide (herein both are collectively referred to as "2A peptide" or interchangeably as "2A site" or "2A cleavage site") which are used by several families of viruses, including Picornavirus, insect viruses, Aphtoviridae, Rotaviruses and Trypanosoma. Best known are 2A sites of rhinovirus and foot-and-mouth disease virus of the Picornaviridae family which are typically used for producing multiple polypeptides from a single ORF.

Accordingly, the term "self-cleavage site" as used herein refers to a cleavage site within the amino acid or nucleotide sequence where this sequence is cleaved or is cleavable without such cleavage involving any additional molecule or where the peptide- or phosphodiesterbond formation in this sequence is prevented in the first place (e.g. through co-translational self-processing as described above).

It is understood that cleavage sites typically comprise several amino acids or are encoded by several codons (e.g. in those cases, wherein the "cleavage site" is not translated into protein but leads to an interruption of translation). Thus, the cleavage site may also serve the purpose of a peptide linker, i.e. sterically separates two peptides. Thus, in some embodiments a "cleavage site" is both a peptide linker and provides above described cleavage function. In this embodiment the cleavage site may encompass additional N-and/or C-terminal amino acids.

In one particular preferred embodiment of the present invention, the self, cleaving site is selected from the group consisting of a viral 2A peptide or 2A-like peptide of Picornavirus, insect viruses, Aphtoviridae, Rotaviruses and Trypanosoma. In one favorable example, the 2A cleavage site is the 2 A peptide of foot and mouth disease virus.

In a preferred embodiment of the present invention, the nucleic acid construct comprised by the first and/or the second vector encodes at least two immunogenic polypeptides, wherein at least one said polypeptides induces a T-cell response and at least one another polypeptide induces a B-cell response.

In a preferred embodiment of the present invention, the ammo acid sequence of the immunogenic polypeptides encoded by the first and second nucleic acid constructs is substantially identical.

In another preferred embodiment of the present invention, at least one of the nucleic acid construct encodes at least one polypeptide selected from the group consisting of (i) the fusion protein F of respiratory syncytial virus (RSV), (ii) nucleoprotein N of RSV and (iii) matrix protein M2 of RSV.

In a specific preferred embodiment of the present invention the nucleic acid constructs comprised by the first and second vector encode the same polypeptide or polypeptides selected from the group consisting of (i) the fusion protein F of respiratory syncytial virus (RSV), (ii) nucleoprotein N of RSV and (iii) matrix protein M2 of RSV. The term "the same polypeptide or polypeptides" refers to polypeptides which are immunologically identical as defined above or have amino acid sequences which are substantially identically as defined above. The term "the same polypeptide or polypeptides" refers to polypeptides having an identical amino acid sequence.

In an specific preferred embodiment of the present invention, at least one nucleic acid construct encodes polypeptides comprising (i) the fusion protein F of respiratory syncytial virus (RSV), (ii) nucleoprotein N of RSV and (iii) matrix protein M2 of RSV. In one favourable embodiment, said nucleic acid construct does not encode any polypeptide in addition to the aforementioned three polypeptides. For example, the vector does not comprise a further nucleic acid construct in addition to the aforementioned nucleic acid construct encoding polypeptides comprising (i) the fusion protein F of respiratory syncytial virus (RSV), (ii) nucleoprotein N of RSV and (iii) matrix protein M2 of RSV.

In one very preferred embodiment of the present invention both nucleic acid constructs encode polypeptides comprising (i) the fusion protein F of respiratory syncytial virus (RSV), (ii) nucleoprotein N of RSV and (iii) matrix protein M2 of RSV. For an example of this embodiment, both nucleic acid constructs do not encode any polypeptide in addition to the aforementioned three polypeptides. For example, both vectors do not comprise a further nucleic acid construct in addition to the aforementioned nucleic acid construct encoding polypeptides comprising (i) the fusion protein F of respiratory syncytial virus (RSV), (ii) nucleoprotein N of RSV and (iii) matrix protein M2 of RSV

### Vaccine

The term "vaccine" refers to a biological preparation which improves immunity to a specific disease. Said preparation may comprise a killed or an attenuated living pathogen. It may also comprise one or more compounds derived from a pathogen suitable for eliciting an immune response. In preferred embodiments of the subject invention, said compound is a polypeptide which is substantially identical or immunologically identical to a polypeptide of said pathogen. Also preferably, the vaccine comprises a nucleic acid construct which encodes an immunogenic polypeptide which is substantially identical or immunologically identical to a polypeptide of said pathogen. In the latter case, it is desired that the polypeptide is expressed in the individual treated with the vaccine. The principle underlying vaccination is the generation of an immunological "memory". Challenging an individual's immune system with a vaccine induces the formation and/or propagation of immune cells which specifically recognize the compound comprised by the vaccine. At least a part of said immune cells remains viable for a period of time which can extend to 10,20 or 30 years after vaccination. If the individual's immune system encounters the pathogen from which the compound capable of eliciting an immune response was derived within the aforementioned period of time, the immune cells generated by vaccination are reactivated and enhance the immune response against the pathogen as compared to the immune response of an individual which has not been challenged with the vaccine and encounters immunogenic compounds of the pathogen for the first time.

### Prime-boost vaccination regimen

In many cases, a single administration of a vaccine is not sufficient to generate the number of long-lasting immune cells which is required for effective protection in case of future infection of the pathogen in question, protect against diseases including tumour diseases or for therapeutically treating a disease, like tumour disease. Consequently, repeated challenge with a biological preparation specific for a specific pathogen or disease is required in order to establish lasting and protective immunity against said pathogen or disease or to cure a given disease. An administration regimen comprising the repeated administration of a vaccine directed against the same pathogen or disease is referred to in the present application as "prime-boost vaccination regimen". Preferably, a prime-boost vaccination regimen involves at least two administrations of a vaccine or vaccine composition directed against a specific pathogen, group of pathogens or diseases. The first administration of the vaccine is referred to as "priming" and any subsequent administration of the same vaccine or a vaccine directed against the same pathogen as the first vaccine can be referred to as "boosting". Thus, in a preferred embodiment of the present invention the prime-boosting vaccination regimen involves one administration of the vaccine for priming the immune response and at least one subsequent administration for boosting the immune response. It is to be understood that 2, 3, 4 or even 5 administrations for boosting the immune response are also contemplated by the present invention.

The period of time between prime and a subsequent administration is, preferably, 1 week, 2 weeks, 4 weeks, 6 weeks or 8 weeks. More preferably, it is 4 weeks. If more than one boost is performed, the subsequent boost is, preferably, administered 1 week, 2 weeks, 4 weeks, 6 weeks or 8 weeks after the preceding boost. For example, the interval is 4 weeks.

The subject or patient to be treated with a prime-boost regimen according to the present invention is, preferably, a mammal or a bird, more preferably a primate, mouse, rat, sheep, goat, cow, pig, horse, goose, chicken, duck or turkey and, most preferably, a human.

Preferably, the use of the vaccine combinations according to the first or second aspect of the present invention will establish protective immunity against a pathogen or disease or will lead to inhibition and/or eradication of infection or a disease caused by infection by the pathogen.

### Vaccine composition

The term "composition" as used in "priming composition" and "boosting composition" refers to the combination of a vector comprising a nucleic acid construct and at least one further compound selected from the group consisting of pharmaceutically acceptable carriers, pharmaceutical excipients and adjuvants. If the boosting composition comprises an immunogenic polypeptide instead of a vector, the boosting composition comprises said at least one immunogenic polypeptide and at least one further compound selected from the group consisting of pharmaceutically acceptable carriers, pharmaceutical excipients and adjuvants.

"Pharmaceutically acceptable" means approved by a regulatory agency of the Federal or a state government or listed in the U.S. Pharmacopeia or other generally recognized pharmacopeia for use in animals, and more particularly in humans.

The term "carrier", as used herein, refers to a pharmacologically inactive substance such as but not limited to a diluent, excipient, or vehicle with which the therapeutically active ingredient is administered. Such pharmaceutical carriers can be liquid or solid. Liquid carrier include but are not limited to sterile liquids, such as saline solutions in water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers, particularly for injectable solutions. A saline solution is a preferred carrier when the pharmaceutical composition is administered intravenously or intranasally by a nebulizer.

Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

Examples of suitable pharmaceutical carriers are described III "Remington's Pharmaceutical Sciences" by E. W. Martin.

The term "adjuvant" refers to agents that augment, stimulate, activate, potentiate, or modulate the immune response to the active ingredient of the composition at either the cellular or humoral level, e.g. immunologic adjuvants stimulate the response of the immune system to the actual antigen, but have no immunological effect themselves. Examples of such adjuvants include but are not limited to inorganic adjuvants (e.g. inorganic metal salts such as aluminium phosphate or aluminium hydroxide), organic adjuvants (e.g. saponins or squalene), oil-based adjuvants (e.g. Freund's complete adjuvant and Freund's incomplete adjuvant), cytokines (e.g. IL-1β, IL-2, IL-7, IL-12, IL-18, GM-CFS, and INF-γ) particulate adjuvants (e.g. immunostimulatory complexes (ISCOMS), liposomes, or biodegradable micro spheres), virosomes, bacterial adjuvants (e.g. monophosphoryl lipid A, or muramyl peptides), synthetic adjuvants (e.g. non-ionic block copolymers, muramyl peptide analogues, or synthetic lipid A), or synthetic polynucleotides adjuvants (e.g polyarginine or polylysine).

A "intranasal administration" is the administration of a vaccine composition of the present invention to the mucosa of the complete respiratory tract including the lung. More preferably, the composition is administered to the mucosa of the nose. Preferably, an intrasal administration is achieved by means of instillation, spray or aerosol. Preferably, said administration does not involve perforation of the mucosa by mechanical means such as a needle.

The term "intramuscular administration" refers to the injection of a vaccine composition into any muscle of an individual. Preferred intramuscular injections are adminsterd into the deltoid, vastus lateralis muscles or the ventrogluteal and dorsogluteal areas.

Surprisingly, it was found that a combination of administration of polynucleotide vectors and proteins provides advantages in the characteristics (e.g., strength) of the vaccination. Therefore, a further aspect the present invention relates to a vaccine combination comprising:
(a) a priming composition comprising, consisting essentially of or consisting of a vector comprising a nucleic acid construct encoding at least one immunogenic polypeptide and
(b) at least one boosting composition comprising, consisting essentially of or consisting of at least one immunogenic polypeptide,
wherein at least one epitope of the immunogenic polypeptide encoded by the nucleic acid construct comprised in the priming composition is immunologically identical to at least one epitope of the immunogenic polypeptide comprised in the boosting composition, for use in a prime-boost vaccination regimen, wherein the priming composition is administered intramuscularly or intranasally and at least one boosting composition is subsequently administered.

In the context of the second aspect of the present invention all terms have the meaning and, where indicated, the preferred meanings defined above regarding the first aspect of the present invention. In particular the term vector, nucleic acid construct, immunogenic polypeptide, intramuscular or intranasal administration, prime boosting vaccination regimen have the above outlined meaning. It is to be understood that the teaching relating to the immunogenic polypeptide is applicable both to immunogenic polypeptide encoded by the nucleic acid of the vector and to the polypeptide, which is administered as such, while the teaching relating to the nucleic acid construct only relates to the nucleic acid comprised in the vector.

It is preferred that the at least one boosting composition is intramuscular or intranasally. Preferably each of the boosting compositions is administered intramuscular or intranasally.

Preferred administration regimens are as follows:
(i) the priming composition is administered intranasally and at least one boosting composition is subsequently administered intramuscularly;
(ii) the priming composition is administered intranasally and at least one boosting composition is subsequently administered intranasally.
(ii) the priming composition is administered intramuscularly and at least one boosting composition is subsequently administered intramuscularly; or
(iv) the priming composition is administered intramuscularly and at least one boosting composition is subsequently administered intranasally, most preferably administration regimen (i) is used.

In a preferred embodiment of this aspect the vector is selected from the group consisting of adenovirus vectors, adeno-associated virus (AA V) vectors (e.g., AA V type 5 and type 2), alphavirus vectors (e.g., Venezuelan equine encephalitis virus (VEE), sindbis virus (SIN), semliki forest virus (SFV), and VEE-SIN chimeras), herpes virus vectors (e.g. vectors derived from cytomegaloviruses, like rhesus cytomegalovirus (RhCMV) (14)), arena virus vectors (e.g. lymphocytic choriomeningitis virus (LCMV) vectors (15)), measles virus vectors, pox virus vectors (e.g., vaccinia virus, modified vaccinia virus Ankara (MV A), NYV AC (derived from the Copenhagen strain of vaccinia), and avipox vectors: canarypox (ALV AC) and fowlpox (FPV) vectors), vesicular stomatitis virus vectors, retrovirus, lentivirus, viral like particles, and bacterial spores.

Highly preferred vectors are adenoviral vectors, in particular adenoviral vectors derived from human or non-human great apes or poxyviral vectors, preferably MV A. Preferred great apes from which the adenoviruses are derived are Chimpanzee *(Pan),* Gorilla *(Gorilla)* and orangutans *(Pongo),* preferably Bonobo *(Pan paniscus)* and common Chimpanzee *(Pan troglodytes).* Typically, naturally occurring non-human great ape adenoviruses are isolated from stool samples of the respective great ape. The most preferred vectors are non-replicating adenoviral vectors based on hAd5, hAd 1 1, hAd26, hAd35, hAd49, ChAd3, ChAd4, ChAd5, ChAd6, ChAd7, ChAd8, ChAd9, ChAd10, ChAd11, ChAd16, ChAd17, ChAd19, ChAd20, ChAd22, ChAd24, ChAd26, ChAd30, ChAd31, ChAd37, ChAd38, ChAd44, ChAd55, ChAd63, ChAd 73, ChAd82, ChAd83 , ChAd146, ChAd147, PanAd1, PanAd2, and PanAd3 vectors or replication-competent Ad4 and Ad7 vectors.

In a preferred embodiment of the present invention the nucleic acid construct comprised by the priming composition has a structure as defined above.

In one preferred embodiment of the present invention, the nucleic acid construct encodes at least the fusion protein F of respiratory syncytial virus (RSV). In a specific example, said nucleic acid construct does not encode any polypeptide in addition to the aforementioned polypeptide. For example, the vector does not comprise a further nucleic acid construct in addition to the aforementioned nucleic acid construct encoding the fusion protein F of respiratory syncytial virus (RSV).

In a specific preferred embodiment of the present invention, the nucleic acid construct encodes polypeptides comprising (i) the fusion protein F of respiratory syncytial virus (RSV), (ii) nucleoprotein N of RSV and (iii) matrix protein M2 of RSV. In an example of such an embodiment, said nucleic acid construct does not encode any polypeptide in addition to the aforementioned three polypeptides. For example, the vector does not comprise a further nucleic acid construct in addition to the aforementioned nucleic acid construct encoding polypeptides comprising (i) the fusion protein F of respiratory syncytial virus (RSV), (ii) nucleoprotein N of RSV and (iii) matrix protein M2 of RSV.

In a preferred embodiment of the present invention, the at least one immunogenic polypeptide comprised by the boosting composition has a structure as defined above. Preferably it is selected from the group consisting of the fusion protein F of respiratory syncytial virus (RSV), (ii) nucleoprotein N of RSV and (iii) matrix protein M2 of RSV or polypeptides having an amino acid sequence which is substantially to the amino acid sequence of the aforementioned polypeptides or polypeptides which are immunologically identically to the aforementioned polypeptides.

In a more preferred embodiment of the present invention, the at least one immunogenic polypeptide comprised by the boosting composition is the fusion protein F of respiratory syncytial virus (RSV). For example, the boosting composition does not comprise immunogenic polypeptides besides said polypeptide (fusion protein F).

In a particularly preferred embodiment of the present invention, the nucleic acid construct encodes (i) the fusion protein F of respiratory syncytial virus (RSV), (ii) nucleoprotein N of RSV and (iii) matrix protein M2 of RSV and the only immunogenic polypeptide comprised by the boosting composition is fusion protein F of RSV.

In one especially preferred embodiment of the present invention, priming of the immune response is performed by intranasal administration of an adenoviral vector (e.g., selected from the list of adenoviral vectors provided herein) and boosting is performed by intramuscular administration of an immunogenic polypeptide. For example, favorably the adenoviral vector can be PanAd3. In this embodiment, the immunogenic polypeptide favorably can be the fusion protein F of RSV and the nucleic acid construct comprised by the vector favourably encodes fusion protein F of RSV, nucleoprotein N of RSV and matrix protein M2 of RSV.

In another especially preferred embodiment of the present invention, priming of the immune response is performed by intranasal administration of an adenoviral vector and boosting is performed by intramuscular administration of a poxviral vector. It is also preferred to use a poxviral vector for priming and an adenoviral vector for boosting of the immune response. For example, favorably the adenoviral vector can be PanAd3 and the poxviral vector can be MV A. In this embodiment, the nucleic acid construct comprised by both vectors encodes, preferably, fusion protein F of RSV, nucleoprotein N of RSV and matrix protein M2 of RSV.

In a further aspect the present invention provides an article of manufacture comprising the vaccine combination according to the first or second aspect of the present invention and an instruction for use.

### Description of the Figures

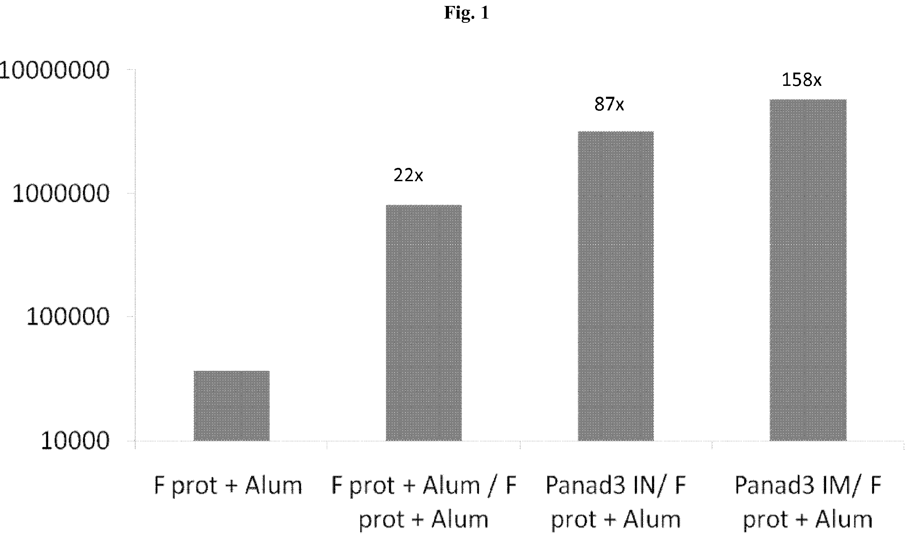

- Fig. 1:: Serum titers of antibodies against F protein measured by Elisa on the recombinant protein F. Titers were determined by serial dilution of pools of sera and represent the dilution that gives a value higher than the background plus 3x the standard deviations. Numbers on the bars represent the fold increase in the antibody titer of the different regimens with respect to a single administration of the recombinant protein

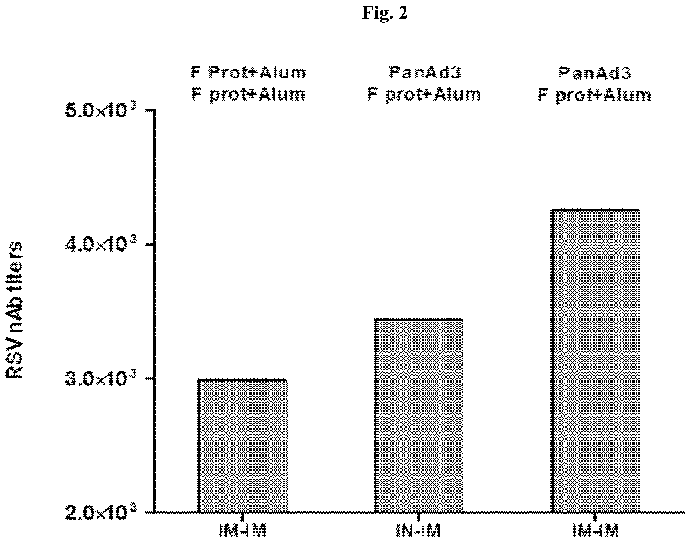

- Fig. 2:: Neutralization titers were measured in a F ACS based RSV infection assay on Hep2 cells using a recombinant RSV-A virus expressing the GFP protein. Data are expressed as EC50 that is the dilution of serum that inhibits viral infection by 50%.

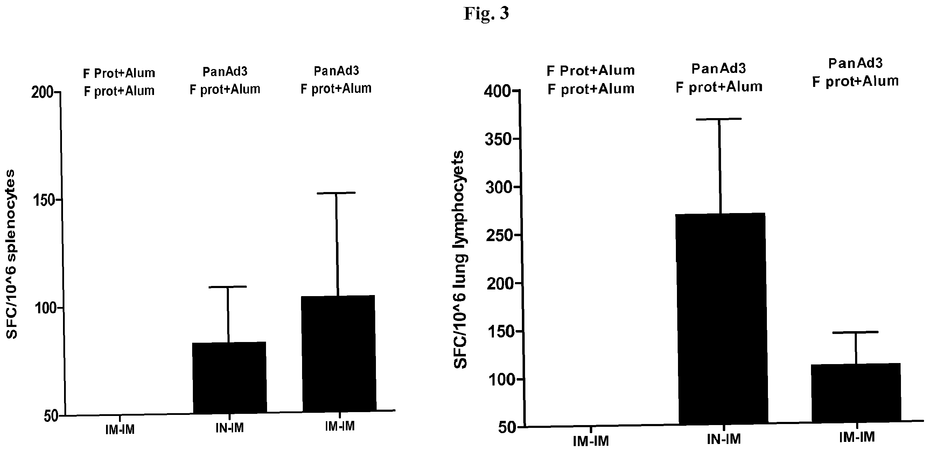

- Fig. 3:: IFNγ T cell Elispot on spleen and on lung lymphocytes after ex-vivo restimulation with peptide pools spanning the whole F protein antigen. Bars represent the average plus standard error of the T cell responses measured in the three groups of animal immunized by the different regimen. Only those animals that have been primed with the PanAd3 vector show T cell responses both in spleen and in lung.

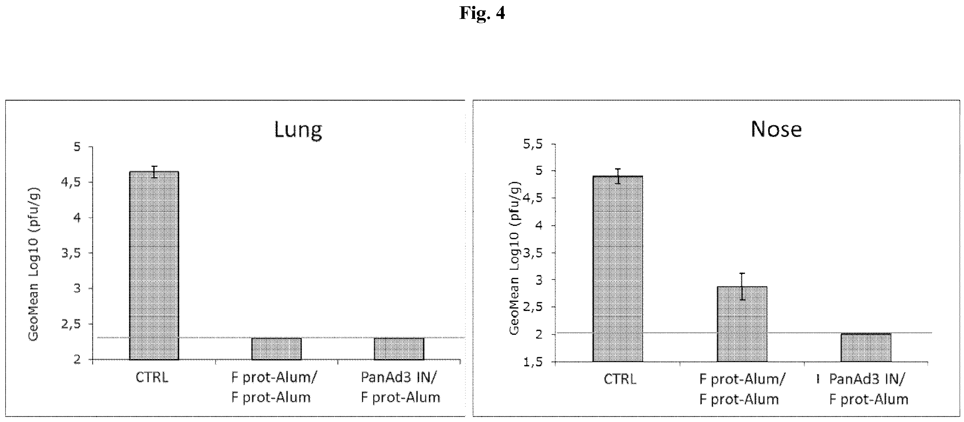

- Fig. 4:: RSV replication in the lung (left panel) and in the nose (right panel) of cotton rats. Virus titer was determined by plaque assay on Hep-2 cells using lysates from the different organs and expressed as the mean of Log10 pfu per gram of tissue. The blue line represents the limit of detection of the assay.

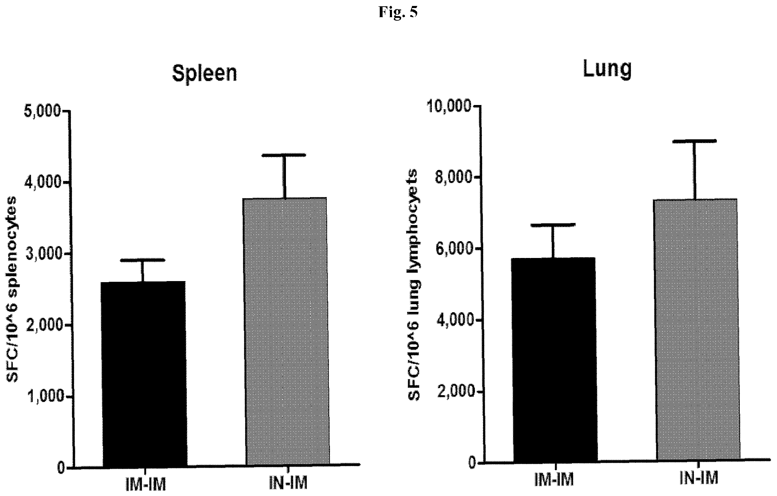

- Fig. 5:: IFNy T cell Elispot on spleen and on lung lymphocytes after ex-vivo restimulation with peptide pools spanning the whole RSV vaccine antigen. Black bars represent the average of the T cell responses measured in the group of animals immunized by PanAd3 in the muscle followed by MVA-RSV in the muscle. Grey bars represent the average plus standard error of the T cell responses measured in the group of animals immunized by PanAd3 in the nose followed by MVA-RSV in the muscle ..

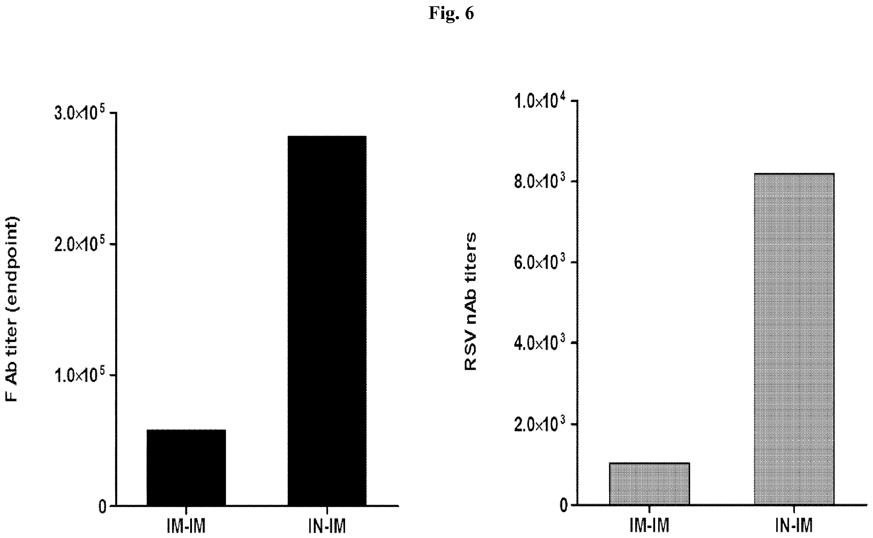

- Fig. 6:: Serum titers (panel A) of antibodies against F protein were measured by ELISA on the recombinant protein F. Neutralization titers (panel B) were measured in a F ACS based RSV infection assay on Hep2 cells using a recombinant RSV -A virus expressing the GFP protein. Data are expressed as EC50 that is the dilution of serum that inhibits viral infection by 50%.

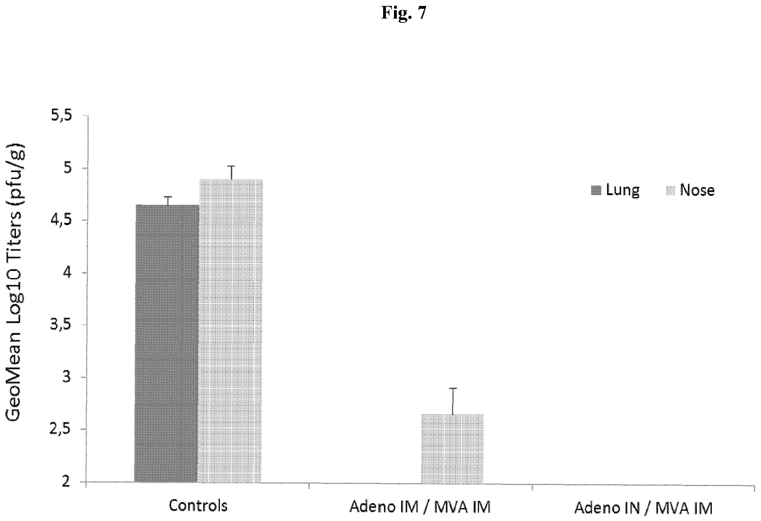

- Fig. 7:: RSV replication in the lungs (dark grey bars) and in the nose (light grey bars) of cotton rats. Virus titer was determined by plaque assay on Hep-2 cells using lysates from the different organs and expressed as the mean of Log 1 0 pfu per gram of tissue

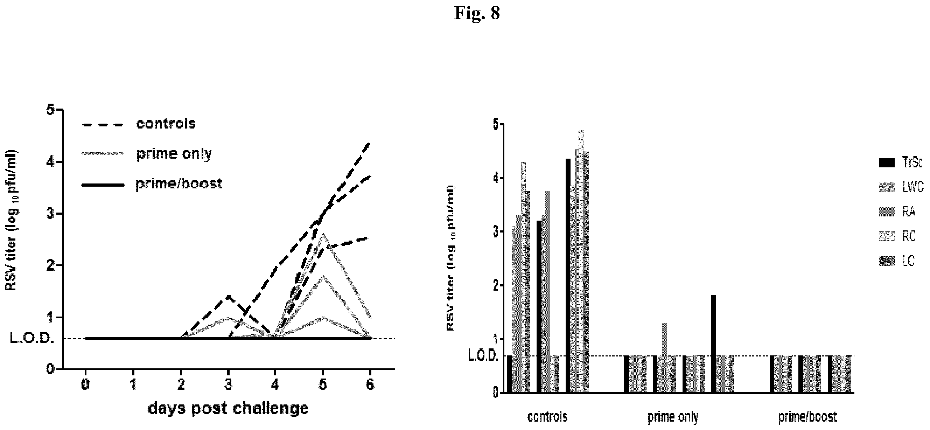

- Fig. 8:: RSV replication in the nasal secretions (left panel) and in the lung (right panel) of infected calves. Virus titer was determined by plaque assay on MDBK cells using nasal swabs or lysates from the different parts of the lung and expressed as the mean of Log 10 pfu per ml of sample. Log 10 = 2 represents the limit of detection of the assay.

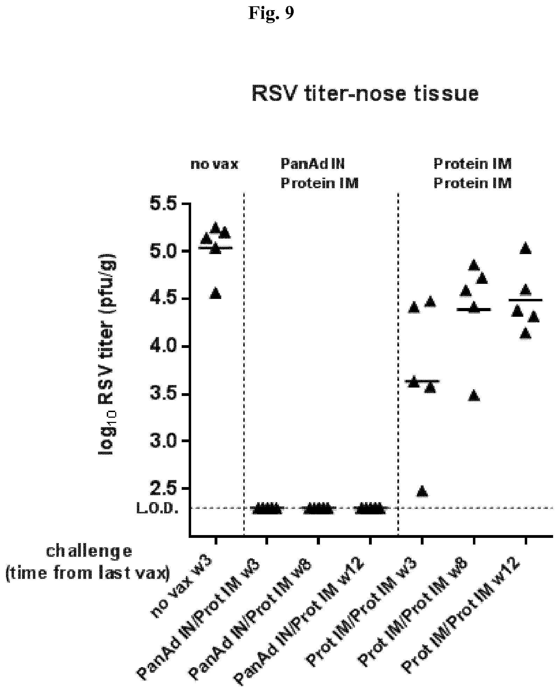

- Fig. 9:: RSV replication in the nose of cotton rats. Virus titer was determined by plaque assay on Hep-2 cells using lysates from te nasal mucosa and expressed as the mean of Log10 pfu per gram of tissue. The dotted line represents the limit of detection of the assay.

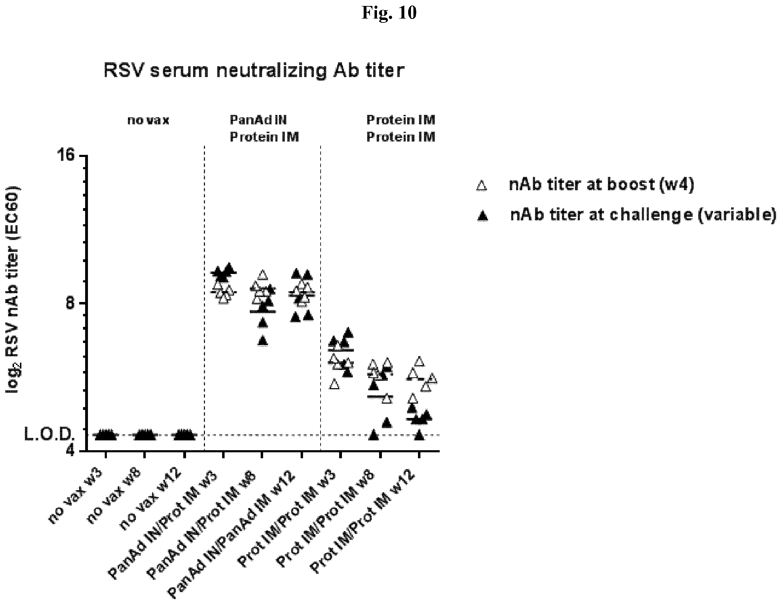

- Fig. 10:: RSV serum neutralizing antibody titers measured at the day of the boost (open triangles = 4 weeks after the prime) and at the day of the challenge (full triangles = 3, 8 and 12 weeks after boost). Neutralization titers were measured by plaque reduction assay on Hep2 cells infected with the human RSV Long strain. Data are expressed as EC60 that is the dilution of serum that inhibits 60% of plaques respect to control.

The following examples are merely intended to illustrate the invention. They shall not limit the scope of the claims in anyway.

### Example 1: Generation ofPanAd3-RSV and MVA-RSV

### Vaccine design

To design the vaccine antigen of the present invention, protein sequences of the F0-, N-, and M2-1-proteins of RSV were retrieved from the National Center for Biotechnology Information (NCBI) RSV Resource database (http://www.ncbi.nlm.nih.gov). Protein sequences were chosen from different RSV subtype A strains.

A F0 consensus sequence was derived by alignment of all non-identical sequences of the F-protein using MUSCLE version 3.6 and applying the majority rule. The vaccine's F0 consensus sequence was designed on the basis of the alignment of the different RSV sequences. The sequence similarity of the vaccine consensus FO sequence was measured performing BLAST analysis, which stands for Basic Local Alignment Search Tool and is publicly available through the NCBI. The highest average similarity of the consensus sequence, calculated compared to all RSV sequences in the database, was 100 % with respect to the human respiratory syncytial virus A2 strain.

Further, the vaccine's F0 sequence lacks the transmembrane region residing in amino acids 525 to 574 to allow for the secretion of F0ΔTM.

Finally, the vaccine F0ΔTM sequence was codon-optimized for expression in eukaryotic cells.

The vaccine's N consensus sequence was derived by alignment of all non-identical sequences of the N-protein using MUSCLE version 3.6 and applying the majority rule. BLAST analysis of the N consensus sequence found the best alignment with the human respiratory syncytial virus A2 strain. The vaccine's N sequence was then codon-optimized for expression in eukaryotic cells.

A M2-1 consensus sequence was derived by alignment of all non-identical sequences of the M2-1-protein using MUSCLE version 3.6 and applying the majority rule. BLAST analysis of the M2-1 consensus sequence found the best alignment with the human respiratory syncytial virus A2 strain. Finally, the vaccine M2-1 sequence was codon-optimized for expression in eukaryotic cells.

The vaccines F0ΔTM sequence and N sequence were spaced by the cleavage sequence 2A of the Foot and Mouth Disease virus. The vaccines N sequence and M2-1 sequence were separated by a flexible linker (GGGSGGG; SEQ ID NO: 6).

Finally, the codon-optimized viral genes were cloned as the single open reading frame F0ΔTM-N-M2-1.

### Generation of DNA plasmids encoding F0ΔTM and F0ΔTM-N-M2-1

Consensus F0ΔTM, N and M2-1 sequences were optimized for mammalian expression, including the addition of a Kozak sequence and codon optimization. The DNA sequence encoding the multi-antigen vaccine was chemically synthesized and then sub-cloned by suitable restriction enzymes EcoRV and Not1 into the pVJTetOCMV shuttle vector under the control of the CMV promoter.

### Generation of PanAd3 viral-vectored RSV vaccine

A viral-vectored RSV vaccine PanAd3/F0ΔTM-N-M2-1 was generated which contains a 809 aa polyprotein (SEQ ID NO.: 7) coding for the consensus F0ΔTM, N and M2-1 proteins fused by a flexible linker.

Bonobo Adenovirus type 3 (PanAd3) is a novel adenovirus strain with improved seroprevalence and has been described previously.

Cloning of F0ΔTM-N-M2-1 from the plasmid vector pVJTetOCMV/F0ΔTM-N-M2-1 into the PanAd3 pre-Adeno vector was performed by cutting out the antigen sequences flanked by homologous regions and enzymatic in vitro recombination.

Cloning of **F0ΔTM-N-M2-1 from** the shuttle plasmid vector p94-F0ΔTM-N-M2-1 into the MVA vector was performed by two steps of enzymatic *in vitro* recombination and selection of the positive recombinant virus by fluorescence microscopy.

### Example 2: Prime with PanAd3-RSV and boost with protein F in mice

### Materials and methods

Groups of 5 *BALB*/*c* mice were immunized with 10^8 vp of PanAd3-RSV by instillation in the nose or by intramuscular injection. Another group was immunized with 5 µg of recombinant protein F (Sino Biologicals Inc. cat n. 11049-V08B) formulated with alumininum hydroxide in the muscle. Four weeks later all animals received 5 µg of recombinant protein F formulated with aluminium hydroxide in the muscle. After four weeks all animals were bled and serum was prepared. A pool of sera of the animals in each group was analyzed by F protein ELISA: Briefly, 96 well microplates were coated with 0.5ug protein F (Sino Biologicals Inc. cat n. 11049-V08B) and incubated with serial dilutions of the sera. After extensive washes, the specific binding was revealed by a secondary anti-mouse IgG antibody conjugated with alkaline phosphatase. Background was determined using *BALB*/*c* pre-immune sera. Antibody titers were expressed as the dilution giving a value equal to background plus 3 times the standard deviation. Neutralizing antibodies were measured by a F ACS-based infection assay. Briefly, a recombinant RSV-A virus expressing GFP (Chen M. et al. J Immunological Methods 2010; 362:180) was used to infect cultured Hep-2 cells for 24 h at a Multiplicity of infection (MOl) giving 20 % infected cells. A serial dilution of pools of mice sera was incubated with the virus 1 hour at 37 °C before addition to the cells. 24 hours later the percentage of infected cells was measured by whole-cell F ACS analysis. Antibody titer was expressed as the serum dilution giving 50 % inhibition of infection (EC50).

T cell responses were measures by IFNγ T cell Elispot: briefly, spleen and lung lymphocytes were plated on 96 well microplates coated with anti-IFNy antibody and stimulated ex-vivo with peptide pools spanning the whole RSV vaccine antigen. After extensive washes, the secreted IFNγ forming a spot on the bottom of the plate was revealed by a secondary antibody conjugated to alkaline phosphatase. The number of spots was counted by an automatic Elispot reader.

### Results

The simian adenovirus PanAd3-RSV containing the RSV antigens F, N and M2-1 was administered to groups of *BALB*/*c* mice either by the intranasal route or by the intramuscular route. A separate group was immunized with the recombinant F protein formulated with aluminium hydroxide by intramuscular injection. Four weeks later, the three groups of mice were boosted with the recombinant F protein formulated with aluminium hydroxide by intramuscular injection. Four weeks after the boost, sera of mice were analyzed by F-protein ELISA and the neutralizing antibody titers were measured by a F ACS based RSV neutralization assay. T cell responses in spleen and lung were measured by IFNγ T cell Elispot.

As shown in Fig. 1, the groups of mice that received PanAd3-RSV as a priming vaccine reached very high levels of anti-F antibody titers in the serum. Priming with PanAd3-RSV increases the antibody titers obtained with a single administration of the F protein by a factor ranging from 87x when Adeno is administered in the nose to 158x when Adeno is administered in the muscle, while two administrations of protein F increase the titer by a factor of 22.

RSV neutralizing antibody titers were measured by a F ACS based cell culture infection assay on Hep2 cells using a recombinant RSV virus expressing GFP. Fig. 2 shows the neutralization titers expressed as the serum dilution which gives 50% of inhibition of infection (EC50). As observed for the anti-F antibody titers, also the neutralizing antibody titer increases in the animals vaccinated by the combination of Adeno prime and protein boost with respect to the protein/protein regimen.

T cell responses were measured in the same groups of mice by IFNγ T-cell Elispot on spleen and lung lymphocytes. As shown in Fig .3 only those groups which were vaccinated with the Adeno vector at prime developed both systemic and local T cell responses. On the contrary, no F specific T cell response was detected in the animals vaccinated with the protein F.

### Example 3: Prime with PanAd3-RSV and boost with protein F in cotton rats

### Materials and Methods

Groups of 5 cotton rats (Sygmoidon Hispidus) were immunized with 10^8 vp of PanAd3RSV by instillation in the nose or with 5ug of recombinant protein F (Sino Biologicals Inc. cat n. 11049-V08B) formulated with Alum hydroxide in the muscle. Four weeks later, all animals received 5 µg of recombinant protein F formulated with aluminum hydroxide in the muscle. After three weeks the two groups of animal plus a control non-vaccinated group, were infected by intranasal administration of 10'''5 pfu of RSV Long strain. Five days after infection all animals were sacrificed and nasal epithelia and lungs were collected and lysed. Serial dilution of the tissue lysates were used to infect cultured Hep2 cells to measure virus titer by counting plaques.

### Results

Two groups of cotton rats were vaccinated by i) Prime and boost with the protein F formulated in aluminum hydroxide or ii) PanAd3-RSV prime in the nose and boost with the protein F formulated in aluminum hydroxide in the muscle. Three weeks after the boost, the animals were challenged by an intranasal administration of 1*Q*/*'5* pfu of RSV Long strain, together with a non-vaccinated control group. Five days after the infection the animal were sacrificed and the virus was titrated by plaque assay on lysates of nasal and lung tissue. As shown in Fig. 4, in the control animals the titer of RSV in the lung and in the nose reached 4-5 log 1 0, while all the animals in the vaccinated groups blocked viral replication in the lung. In contrast, only those animals that received the combination of Adeno and protein showed complete sterilizing immunity also in the upper respiratory tract.

### Example 4: Longevity of neutralizing antibodies against RSV after prime with PanAd3-RSV and boost with protein F in cotton rats

### Materials and Methods

Groups of 5 cotton rats (Sygmoidon Hispidus) were immunized with 10^8 vp of PanAd3RSV by instillation in the nose or with 5ug of recombinant protein F (Sino Biologicals Inc. cat n.11049-V08B) formulated with Alum hydroxide in the muscle. Four weeks later, all animals received 5µg of recombinant protein F formulated with Alum hydroxide in the muscle. After three weeks the two groups of animal plus a control non vaccinated group, were infected by intranasal administration of 10^5 pfu of RSV Long strain. Five days after infection all animals were sacrificed and nasal epithelia and lungs were collected and lysed. Serial dilutions of the tissue lysates were used to infect cultured Hep2 cells to measure virus titer by counting plaques. Serum neutralizing antibodies were measured by plaque reduction assay in Hep2 cells infected with RSV Long strain. Titer was expressed as the serum dilution giving 60% reduction of plaque respect to not inhibited controls..

### Results

Two groups of cotton rats were vaccinated by i) PanAd3-RSV prime in the nose and boost with the protein F formulated in Alum Hydroxide in the muscle or ii) Prime and boost with the protein F formulated in Alum Hydroxide. At three, eight and twelve weeks after the boost, the animals were challenged by an intranasal administration of 10^5 pfu of RSV Long strain, together with a non vaccinated control group. Five days after the infection the animal were sacrificed and the virus was titrated by plaque assay on lysates of nasal and lung tissue. As shown in Fig 9, in the control animals the titer of RSV in the nose reached 4-5 10glO, while only those animals that received the combination of Adeno and protein showed complete sterilizing immunity in the upper respiratory tract. Serum neutralizing antibodies were measured at the day of the boost (4 weeks after the prime) and at the day of the challenge, which was at 3,8 and 12 weeks after the boost. As shown in Fig 10, the neutralizing titers remained high and sustained only in those group that were vaccinated with the combination of Adeno and protein, while the neutralizing titers of those vaccinated with the protein slowly decayed over time.

### Example 5: T-cell response after intranasal prime with PanAd3-RSV and boost with MVA-RSV

### Materials and methods

10⁸ virus particles (vp) of PanAd3-RSV containing the RSV antigens F, N and M2-1 were administered to groups of 10 CD 1 mice by instillation in the nose or by the intramuscular route. Four weeks later, all animals received in the muscle 10⁷ plaque forming units (pfu) of MVARSV containing the RSV antigens F, N and M2-1. After four weeks, the animals were sacrificed, lymphocytes were isolated from the spleen and the lung and serum from blood was prepared. T cell responses, titers of anti-F antibodies and RSV neutralizing antibodies were measured as described above.

### Results

A heterologous prime/boost vaccination regimen based on administering PanAd3-RSV in the nose at prime and boosting 4 weeks later with MVA-RSV in the muscle was compared to a regimen based on PanAd3-RSV prime and MVA-RSV boost, both administered in the muscle in outbred CD 1 mice. Four weeks after MV A boost, the mice were sacrificed and the RSV specific T cell responses were measured in the spleen and in the lung. As shown in Fig.5 PanAd3-RSV administration in the nose at prime elicited stronger IFN-y T cell responses both in the spleen and in the lung.

The improvement in the immune response after Adeno prime in the nose was confirmed by the increase of antibody against the F protein (Fig. 7, panel A) and of neutralizing antibody titers in the sera (Fig. 7, panel B).

### Example 6: Immunity in cotton rats after prime with PanAd3-RSV and boost with MVA-RSV

### Materials and methods

Groups of 5 cotton rats (Sygmoidon Hispidus) were immunized with 10⁸ vp of PanAd3RSV by instillation in the nose or by intramuscular injection. Four weeks later all animals received 10⁷ pfu of MVA-RSV in the muscle. After three weeks the two groups of animal plus a control non vaccinated group, were infected by intranasal administration of 10 pfu of RSV Long strain. Five days after infection all animals were sacrificed and nasal epithelia and lungs were collected and lysed. Serial dilution of the tissue lysates were used to infect cultured Hep2 cells to measure virus titer by counting plaques.

### Results

Two groups of cotton rats were vaccinated by heterologous prime/boost with PanAd3-RSV/MVA-RSV to compare the difference between priming with PanAd3-RSV in the nose or in the muscle. Both groups were boosted with MV A in the muscle at 4 weeks interval. A third group of non vaccinated animal was used as a control. Three weeks after the boost, the animals were challenged by an intranasal administration of 10 pfu of RSV Long strain. Five days after the infection the animals were sacrificed and the virus was titrated by plaque assay on lysates of nasal and lung tissue. As shown in Fig. 7, in the control animals the titer of RSV in the lung and in the nose reached 4-5 log 1 0, while all the animals in the vaccinated groups blocked viral replication in the lung. In contrast, only those animals that received Adeno in the nose at prime showed complete sterilizing immunity also in the upper respiratory tract.

### Example 7: Immunity in cattle after prime with PanAd3-RSV and boost with MVA-RSV as compared to vaccination with PanAd3-RSV alone

### Materials and Methods

Two groups (A and B) of 3 and 4 newborn (2-4 weeks old) seronegative calves (screened by BRSV plaque reduction assay) were immunized with 5x10^10 vp of PanAd3-RSV by nasal delivery via a spray device. Eight weeks after prime, group B received 2x10^8 pfu of MVA-RSV in the muscle. A third group, group C, was not vaccinated and used as a control group. Four weeks after prime (for group A) or after boost (for group B) the two groups of animals plus the control group C, were infected by intranasal and intratracheal administration of 10^4 pfu of BRSV Snook strain. Six days after infection all animals were sacrificed. Nasal secretions were collected by nasal swabs every day during the infection. At sacrifice, tracheal scrape and lung washes were collected plus section of different parts of the lung (right apical lobe, right cardiac lobe, left cardiac lobe) which were lysed in appropriate buffer. Serial dilution of the tissue lysates were used to infect cultured bovine MDBK cells in order to measure virus titer by counting plaques.

### Results

Two groups of 2-4 weeks old seronegative calves were vaccinated by i) single intranasal administration of PanAd3-RSV or ii) intranasal prime with PanAd3-RSV followed by intramuscular MVA-RSV boost 8 weeks later. The animals were challenged four weeks after vaccination by intranasal and intratracheal administration of 10⁴pfu of BRSV Snook strain. Six days after the infection, when the virus replication peaks in the lung and in the nose causing maximal pulmonary pathology, the animals were sacrificed. Virus titer in nasal secretions was determined throughout the course of infection by plaque assay on MDBK cells, while it was measured in the lung at the day of sacrifice. The results in Fig. 8, panel B, clearly indicate that the group that received only one dose of PanAd3-RSV in the nose was able to blunt viral replication in the lung almost completely. Administration of PanAd3-RSV in the nose led to a reduced and transient level of peak virus load in nasal secretion with respect to control animals (Fig. 9 panel A). The group that received PanAd3-RSV in the nose followed by MVA-RSV in the muscle showed sterilizing immunity to the virus both in the upper and in the lower respiratory tract (Fig.9).

### Conclusions:

The combination of a PanAd3-RSV (IN) and recombinant protein (IM) induced stronger and longer lasting immunity (Examples 3 and 4) as compared to homologous regimens with two 1M administrations of recombinant protein F. It could also be shown in mice that stronger immune responses were generated by the combination of IN prime with PanAd3-RSV and IM boost with recombinant protein F. Thus, priming of an immune response with a vector-based vaccine improves the efficacy of a boost with a peptide vaccine as compared to priming with a peptide vaccine.

If heterologous prime/boost vaccination regimens with adenoviral vectors and poxviral vectors are employed, the combination of an intranasal prime and intramuscular boost elicited a stronger immune response than intramuscular prime and intramuscular boost as shown in example 5 for mice and example 6 for cotton rats. Thus, heterologous prime/boost vaccination regimens can be optimized by careful selections of the routes of administration of the two vaccines in order to achieve the best immunization.

## Claims

1. A vaccine combination comprising:
a) a priming composition comprising a first vector comprising a nucleic acid construct encoding at least one immunogenic polypeptide and
b) at least one boosting composition comprising a second vector comprising a nucleic acid construct encoding at least one immunogenic polypeptide,
wherein at least one epitope of the immunogenic polypeptide encoded by the nucleic acid construct comprised in the first vector is immunologically identical to at least one epitope of the immunogenic polypeptide encoded by the nucleic acid construct comprised in the second vector,
for use in a prime-boost vaccination regimen, wherein:
(i) the priming composition is administered intranasally and at least one boosting composition is subsequently administered intramuscularly;
(ii) the priming composition is administered intranasally and at least one boosting composition is subsequently administered intranasally.
(iii) the priming composition is administered intramuscularly and at least one boosting composition is subsequently administered intramuscularly; or
(iv) the priming composition is administered intramuscularly and at least one boosting composition is subsequently administered intranasally.

2. The vaccine combination of claim 1, wherein the first vector is an adenoviral vector.

3. The vaccine combination of claim 2, wherein the adenoviral vector is a non-human great ape-derived adenoviral vector, preferably a chimpanzee or bonobo adenoviral vector.

4. The vaccine combination of any one of claims 1 to 3, wherein the second vector is a poxviral vector, preferably MVA or an adenoviral vector.

5. The vaccine combination of claim 4, wherein the adenoviral vector comprising the first nucleic acid construct is immunologically different from the adenoviral vector comprising the second nucleic acid construct.

6. The vaccine combination according to any one of claims 1 to 5, wherein the first and/or the second nucleic acid construct encode at least two polypeptides.

7. The vaccine combination according to claim 6, wherein one of the polypeptides induces a T-cell response and another polypeptide induces a B-cell response.

8. The vaccine combination of claim 6 or 7, wherein the at least two polypeptides encoded by the first and/or second nucleic acid construct are linked by cleavage site.

9. The vaccine combination of claim 8, wherein the cleavage site is a self-cleaving site or an endopeptidase cleavage site.

10. The vaccine combination of claim 9, wherein the self-cleaving site is a 2A cleavage site selected from the group consisting of a viral 2A peptide or 2A-like peptide of Picornavirus, insect viruses, Aphtoviridae, Rotaviruses and Trypanosoma, preferably wherein the 2A cleavage site is the 2 A peptide of foot and mouth disease virus.

11. The vaccine combination of claims 1 to 10, wherein the amino acid sequence of the immunogenic polypeptides encoded by the first and second nucleic acid constructs is substantially identical.

12. The vaccine combination according to any one of claims 1 to 11, wherein at least one nucleic acid construct encodes polypetides comprising (i) the fusion protein F of respiratory syncytial virus (RSV), (ii) nucleoprotein N of RSV and (iii) matrix protein M2 of RSV.

13. The vaccine combination according to claim 12, wherein the first and the second nucleic acid construct encode polypetides comprising (i) the fusion protein F of RSV, (ii) nucleoprotein N of RSV and (iii) matrix protein M2 of RSV.

14. The vaccine combination of any of claims 1 to 13, wherein
(i) the first vector is an adenoviral vector and the second vector is a poxviral vector; or The first vector is a poxviral vector and the second vector is an adenoviral vector; and
(ii) the first and the second nucleic acid construct encode polypetides comprising (i) the fusion protein F of RSV, (ii) nucleoprotein N of RSV and (iii) matrix protein M2 of RSV.

15. The vaccine combination of claim 14, wherein the priming composition is administered intranasally and at least one boosting composition is subsequently administered intramuscularly; or the priming composition is administered intranasally and at least one boosting composition is subsequently administered intranasally.

16. A vaccine combination comprising:
a) a priming composition comprising a vector comprising a nucleic acid construct encoding at least one immunogenic polypeptide and
b) at least one boosting composition comprising at least one immunogenic polypeptide, wherein at least one epitope of the immunogenic polypeptide encoded by the nucleic acid construct comprised in the priming composition is immunologically identical to at least one epitope of the immunogenic polypeptide comprised in the boosting composition,
for use in a prime-boost vaccination regimen, wherein the priming composition is administered intramuscular or intranasally and at least one boosting composition is subsequently administered.

17. The vaccine combination according to claim 16, wherein the administration of at least one boosting composition is intramuscular or intranasally.

18. The combination according to claim 16 or 17, wherein
(i) the priming composition is administered intranasally and at least one boosting composition is subsequently administered intramuscularly;
(ii) the priming composition is administered intranasally and at least one boosting composition is subsequently administered intranasally.
(iii) the priming composition is administered intramuscularly and at least one boosting composition is subsequently administered intramuscularly; or
(iv) the priming composition is administered intramuscularly and at least one boosting composition is subsequently administered intranasally.

19. The vaccine combination according to any of claims 16 to 18, wherein the vector is an adenoviral vector.

20. The vaccine combination according to claim 19, wherein the adenoviral vector is a non-human great ape-derived adenoviral vector, preferably, a chimpanzee or bonobo adenoviral vector.

21. The vaccine combination according to any one of claims 16 to 20, wherein the nucleic acid construct encodes at least two polypeptides.

22. The vaccine combination according to any one of claims 16 to 21, wherein one of the polypeptides induces a T-cell response and another polypeptide induces a B-cell response.

23. The vaccine combination of claim 22, wherein the cleavage site is a self-cleaving site or an endopeptidase cleavage site.

24. The vaccine combination according to any of claims 21 to 23, wherein the nucleic acid construct encodes polypeptides comprising (i) the fusion protein F of RSV, (ii) nucleoprotein N of RSV and (iii) matrix protein M2 of RSV.

25. The vaccine combination according to any one of claims 16 to 24, wherein the polypeptide for boosting an immune response is fusion protein F of RSV.

26. The vaccine combination according to claims 6 to 13 and 22 to 25, for enhancing the B cell response against a polypeptide, which induces a B-cell response.


SEQUENCE LISTING

<110> Okairos AG, et al.

<120> Novel prime-boosting regimens involving immunogenic polypeptides
encoded by polynucleotides

<130> 578-49PCT2

<150> PCT/EP2012/063196
<151> 2012-07-05

<160> 7

<170> BiSSAP 1.0

<210> 1
<211> 360
<212> PRT
<213> Respiratory syncytial virus

<220>
<221> SOURCE
<222> 1..360
<223> /mol_type="protein"
      /organism="Respiratory syncytial virus"

<400> 1
Val Leu His Leu Glu Gly Glu Val Asn Lys Ile Lys Ser Ala Leu Leu
1               5                   10                  15
Ser Thr Asn Lys Ala Val Val Ser Leu Ser Asn Gly Val Ser Val Leu
            20                  25                  30
Thr Ser Lys Val Leu Asp Leu Lys Asn Tyr Ile Asp Lys Gln Leu Leu
        35                  40                  45
Pro Ile Val Asn Lys Gln Ser Cys Ser Ile Ser Asn Ile Glu Thr Val
    50                  55                  60
Ile Glu Phe Gln Gln Lys Asn Asn Arg Leu Leu Glu Ile Thr Arg Glu
65                  70                  75                  80
Phe Ser Val Asn Ala Gly Val Thr Thr Pro Val Ser Thr Tyr Met Leu
                85                  90                  95
Thr Asn Ser Glu Leu Leu Ser Leu Ile Asn Asp Met Pro Ile Thr Asn
            100                 105                 110
Asp Gln Lys Lys Leu Met Ser Asn Asn Val Gln Ile Val Arg Gln Gln
        115                 120                 125
Ser Tyr Ser Ile Met Ser Ile Ile Lys Glu Glu Val Leu Ala Tyr Val
    130                 135                 140
Val Gln Leu Pro Leu Tyr Gly Val Ile Asp Thr Pro Cys Trp Lys Leu
145                 150                 155                 160
His Thr Ser Pro Leu Cys Thr Thr Asn Thr Lys Glu Gly Ser Asn Ile
                165                 170                 175
Cys Leu Thr Arg Thr Asp Arg Gly Trp Tyr Cys Asp Asn Ala Gly Ser
            180                 185                 190
Val Ser Phe Phe Pro Gln Ala Glu Thr Cys Lys Val Gln Ser Asn Arg
        195                 200                 205
Val Phe Cys Asp Thr Met Asn Ser Leu Thr Leu Pro Ser Glu Val Asn
    210                 215                 220
Leu Cys Asn Val Asp Ile Phe Asn Pro Lys Tyr Asp Cys Lys Ile Met
225                 230                 235                 240
Thr Ser Lys Thr Asp Val Ser Ser Ser Val Ile Thr Ser Leu Gly Ala
                245                 250                 255
Ile Val Ser Cys Tyr Gly Lys Thr Lys Cys Thr Ala Ser Asn Lys Asn
            260                 265                 270
Arg Gly Ile Ile Lys Thr Phe Ser Asn Gly Cys Asp Tyr Val Ser Asn

275                 280                 285
Lys Gly Val Asp Thr Val Ser Val Gly Asn Thr Leu Tyr Tyr Val Asn
    290                 295                 300
Lys Gln Glu Gly Lys Ser Leu Tyr Val Lys Gly Glu Pro Ile Ile Asn
305                 310                 315                 320
Phe Tyr Asp Pro Leu Val Phe Pro Ser Asp Glu Phe Asp Ala Ser Ile
                325                 330                 335
Ser Gln Val Asn Glu Lys Ile Asn Gln Ser Leu Ala Phe Ile Arg Lys
            340                 345                 350
Ser Asp Glu Leu Leu His Asn Val
        355                 360

<210> 2
<211> 524
<212> PRT
<213> Respiratory syncytial virus

<220>
<221> SOURCE
<222> 1..524
<223> /mol_type="protein"
      /organism="Respiratory syncytial virus"

<400> 2
Met Glu Leu Leu Ile Leu Lys Ala Asn Ala Ile Thr Thr Ile Leu Thr
1               5                   10                  15
Ala Val Thr Phe Cys Phe Ala Ser Gly Gln Asn Ile Thr Glu Glu Phe
            20                  25                  30
Tyr Gln Ser Thr Cys Ser Ala Val Ser Lys Gly Tyr Leu Ser Ala Leu
        35                  40                  45
Arg Thr Gly Trp Tyr Thr Ser Val Ile Thr Ile Glu Leu Ser Asn Ile
    50                  55                  60
Lys Glu Asn Lys Cys Asn Gly Thr Asp Ala Lys Val Lys Leu Ile Lys
65                  70                  75                  80
Gln Glu Leu Asp Lys Tyr Lys Asn Ala Val Thr Glu Leu Gln Leu Leu
                85                  90                  95
Met Gln Ser Thr Pro Ala Thr Asn Asn Arg Ala Arg Arg Glu Leu Pro
            100                 105                 110
Arg Phe Met Asn Tyr Thr Leu Asn Asn Ala Lys Lys Thr Asn Val Thr
        115                 120                 125
Leu Ser Lys Lys Arg Lys Arg Arg Phe Leu Gly Phe Leu Leu Gly Val
    130                 135                 140
Gly Ser Ala Ile Ala Ser Gly Val Ala Val Ser Lys Val Leu His Leu
145                 150                 155                 160
Glu Gly Glu Val Asn Lys Ile Lys Ser Ala Leu Leu Ser Thr Asn Lys
                165                 170                 175
Ala Val Val Ser Leu Ser Asn Gly Val Ser Val Leu Thr Ser Lys Val
            180                 185                 190
Leu Asp Leu Lys Asn Tyr Ile Asp Lys Gln Leu Leu Pro Ile Val Asn
        195                 200                 205
Lys Gln Ser Cys Ser Ile Ser Asn Ile Glu Thr Val Ile Glu Phe Gln
    210                 215                 220
Gln Lys Asn Asn Arg Leu Leu Glu Ile Thr Arg Glu Phe Ser Val Asn
225                 230                 235                 240
Ala Gly Val Thr Thr Pro Val Ser Thr Tyr Met Leu Thr Asn Ser Glu
                245                 250                 255
Leu Leu Ser Leu Ile Asn Asp Met Pro Ile Thr Asn Asp Gln Lys Lys
            260                 265                 270
Leu Met Ser Asn Asn Val Gln Ile Val Arg Gln Gln Ser Tyr Ser Ile
        275                 280                 285
Met Ser Ile Ile Lys Glu Glu Val Leu Ala Tyr Val Val Gln Leu Pro
    290                 295                 300
Leu Tyr Gly Val Ile Asp Thr Pro Cys Trp Lys Leu His Thr Ser Pro

305                 310                 315                 320
Leu Cys Thr Thr Asn Thr Lys Glu Gly Ser Asn Ile Cys Leu Thr Arg
                325                 330                 335
Thr Asp Arg Gly Trp Tyr Cys Asp Asn Ala Gly Ser Val Ser Phe Phe
            340                 345                 350
Pro Gln Ala Glu Thr Cys Lys Val Gln Ser Asn Arg Val Phe Cys Asp
        355                 360                 365
Thr Met Asn Ser Leu Thr Leu Pro Ser Glu Val Asn Leu Cys Asn Val
    370                 375                 380
Asp Ile Phe Asn Pro Lys Tyr Asp Cys Lys Ile Met Thr Ser Lys Thr
385                 390                 395                 400
Asp Val Ser Ser Ser Val Ile Thr Ser Leu Gly Ala Ile Val Ser Cys
                405                 410                 415
Tyr Gly Lys Thr Lys Cys Thr Ala Ser Asn Lys Asn Arg Gly Ile Ile
            420                 425                 430
Lys Thr Phe Ser Asn Gly Cys Asp Tyr Val Ser Asn Lys Gly Val Asp
        435                 440                 445
Thr Val Ser Val Gly Asn Thr Leu Tyr Tyr Val Asn Lys Gln Glu Gly
    450                 455                 460
Lys Ser Leu Tyr Val Lys Gly Glu Pro Ile Ile Asn Phe Tyr Asp Pro
465                 470                 475                 480
Leu Val Phe Pro Ser Asp Glu Phe Asp Ala Ser Ile Ser Gln Val Asn
                485                 490                 495
Glu Lys Ile Asn Gln Ser Leu Ala Phe Ile Arg Lys Ser Asp Glu Leu
            500                 505                 510
Leu His Asn Val Asn Ala Gly Lys Ser Thr Thr Asn
        515                 520

<210> 3
<211> 170
<212> PRT
<213> Respiratory syncytial virus

<220>
<221> SOURCE
<222> 1..170
<223> /mol_type="protein"
      /organism="Respiratory syncytial virus"

<400> 3
Ile Lys Ile Leu Arg Asp Ala Gly Tyr His Val Lys Ala Asn Gly Val
1               5                   10                  15
Asp Val Thr Thr His Arg Gln Asp Ile Asn Gly Lys Glu Met Lys Phe
            20                  25                  30
Glu Val Leu Thr Leu Ala Ser Leu Thr Thr Glu Ile Gln Ile Asn Ile
        35                  40                  45
Glu Ile Glu Ser Arg Lys Ser Tyr Lys Lys Met Leu Lys Glu Met Gly
    50                  55                  60
Glu Val Ala Pro Glu Tyr Arg His Asp Ser Pro Asp Cys Gly Met Ile
65                  70                  75                  80
Ile Leu Cys Ile Ala Ala Leu Val Ile Thr Lys Leu Ala Ala Gly Asp
                85                  90                  95
Arg Ser Gly Leu Thr Ala Val Ile Arg Arg Ala Asn Asn Val Leu Lys
            100                 105                 110
Asn Glu Met Lys Arg Tyr Lys Gly Leu Leu Pro Lys Asp Ile Ala Asn
        115                 120                 125
Ser Phe Tyr Glu Val Phe Glu Lys Tyr Pro His Phe Ile Asp Val Phe
    130                 135                 140
Val His Phe Gly Ile Ala Gln Ser Ser Thr Arg Gly Gly Ser Arg Val
145                 150                 155                 160
Glu Gly Ile Phe Ala Gly Leu Phe Met Asn
                165                 170

<210> 4
<211> 391
<212> PRT
<213> Respiratory syncytial virus

<220>
<221> SOURCE
<222> 1..391
<223> /mol_type="protein"
      /organism="Respiratory syncytial virus"

<400> 4
Met Ala Leu Ser Lys Val Lys Leu Asn Asp Thr Leu Asn Lys Asp Gln
1               5                   10                  15
Leu Leu Ser Ser Ser Lys Tyr Thr Ile Gln Arg Ser Thr Gly Asp Ser
            20                  25                  30
Ile Asp Thr Pro Asn Tyr Asp Val Gln Lys His Ile Asn Lys Leu Cys
        35                  40                  45
Gly Met Leu Leu Ile Thr Glu Asp Ala Asn His Lys Phe Thr Gly Leu
    50                  55                  60
Ile Gly Met Leu Tyr Ala Met Ser Arg Leu Gly Arg Glu Asp Thr Ile
65                  70                  75                  80
Lys Ile Leu Arg Asp Ala Gly Tyr His Val Lys Ala Asn Gly Val Asp
                85                  90                  95
Val Thr Thr His Arg Gln Asp Ile Asn Gly Lys Glu Met Lys Phe Glu
            100                 105                 110
Val Leu Thr Leu Ala Ser Leu Thr Thr Glu Ile Gln Ile Asn Ile Glu
        115                 120                 125
Ile Glu Ser Arg Lys Ser Tyr Lys Lys Met Leu Lys Glu Met Gly Glu
    130                 135                 140
Val Ala Pro Glu Tyr Arg His Asp Ser Pro Asp Cys Gly Met Ile Ile
145                 150                 155                 160
Leu Cys Ile Ala Ala Leu Val Ile Thr Lys Leu Ala Ala Gly Asp Arg
                165                 170                 175
Ser Gly Leu Thr Ala Val Ile Arg Arg Ala Asn Asn Val Leu Lys Asn
            180                 185                 190
Glu Met Lys Arg Tyr Lys Gly Leu Leu Pro Lys Asp Ile Ala Asn Ser
        195                 200                 205
Phe Tyr Glu Val Phe Glu Lys Tyr Pro His Phe Ile Asp Val Phe Val
    210                 215                 220
His Phe Gly Ile Ala Gln Ser Ser Thr Arg Gly Gly Ser Arg Val Glu
225                 230                 235                 240
Gly Ile Phe Ala Gly Leu Phe Met Asn Ala Tyr Gly Ala Gly Gln Val
                245                 250                 255
Met Leu Arg Trp Gly Val Leu Ala Lys Ser Val Lys Asn Ile Met Leu
            260                 265                 270
Gly His Ala Ser Val Gln Ala Glu Met Glu Gln Val Val Glu Val Tyr
        275                 280                 285
Glu Tyr Ala Gln Lys Leu Gly Gly Glu Ala Gly Phe Tyr His Ile Leu
    290                 295                 300
Asn Asn Pro Lys Ala Ser Leu Leu Ser Leu Thr Gln Phe Pro His Phe
305                 310                 315                 320
Ser Ser Val Val Leu Gly Asn Ala Ala Gly Leu Gly Ile Met Gly Glu
                325                 330                 335
Tyr Arg Gly Thr Pro Arg Asn Gln Asp Leu Tyr Asp Ala Ala Lys Ala
            340                 345                 350
Tyr Ala Glu Gln Leu Lys Glu Asn Gly Val Ile Asn Tyr Ser Val Leu
        355                 360                 365
Asp Leu Thr Ala Glu Glu Leu Glu Ala Ile Lys His Gln Leu Asn Pro
    370                 375                 380
Lys Asp Asn Asp Val Glu Leu
385                 390

<210> 5
<211> 194
<212> PRT
<213> Respiratory syncytial virus

<220>
<221> SOURCE
<222> 1..194
<223> /mol_type="protein"
      /organism="Respiratory syncytial virus"

<400> 5
Met Ser Arg Arg Asn Pro Cys Lys Phe Glu Ile Arg Gly His Cys Leu
1               5                   10                  15
Asn Gly Lys Arg Cys His Phe Ser His Asn Tyr Phe Glu Trp Pro Pro
            20                  25                  30
His Ala Leu Leu Val Arg Gln Asn Phe Met Leu Asn Arg Ile Leu Lys
        35                  40                  45
Ser Met Asp Lys Ser Ile Asp Thr Leu Ser Glu Ile Ser Gly Ala Ala
    50                  55                  60
Glu Leu Asp Arg Thr Glu Glu Tyr Ala Leu Gly Val Val Gly Val Leu
65                  70                  75                  80
Glu Ser Tyr Ile Gly Ser Ile Asn Asn Ile Thr Lys Gln Ser Ala Cys
                85                  90                  95
Val Ala Met Ser Lys Leu Leu Thr Glu Leu Asn Ser Asp Asp Ile Lys
            100                 105                 110
Lys Leu Arg Asp Asn Glu Glu Leu Asn Ser Pro Lys Ile Arg Val Tyr
        115                 120                 125
Asn Thr Val Ile Ser Tyr Ile Glu Ser Asn Arg Lys Asn Asn Lys Gln
    130                 135                 140
Thr Ile His Leu Leu Lys Arg Leu Pro Ala Asp Val Leu Lys Lys Thr
145                 150                 155                 160
Ile Lys Asn Thr Leu Asp Ile His Lys Ser Ile Thr Ile Asn Asn Pro
                165                 170                 175
Lys Glu Ser Thr Val Ser Asp Thr Asn Asp His Ala Lys Asn Asn Asp
            180                 185                 190
Thr Thr


<210> 6
<211> 7
<212> PRT
<213> Artificial Sequence

<220>
<221> SOURCE
<222> 1..7
<223> /mol_type="protein"
      /note="peptide linker"
      /organism="Artificial Sequence"

<400> 6
Gly Gly Gly Ser Gly Gly Gly
1               5

<210> 7
<211> 1146
<212> PRT
<213> Artificial Sequence

<220>
<221> SOURCE
<222> 1..1146

<223> /mol_type="protein"
      /note="immunogenic polyprotein"
      /organism="Artificial Sequence"

<400> 7
Met Glu Leu Leu Ile Leu Lys Ala Asn Ala Ile Thr Thr Ile Leu Thr
1               5                   10                  15
Ala Val Thr Phe Cys Phe Ala Ser Gly Gln Asn Ile Thr Glu Glu Phe
            20                  25                  30
Tyr Gln Ser Thr Cys Ser Ala Val Ser Lys Gly Tyr Leu Ser Ala Leu
        35                  40                  45
Arg Thr Gly Trp Tyr Thr Ser Val Ile Thr Ile Glu Leu Ser Asn Ile
    50                  55                  60
Lys Glu Asn Lys Cys Asn Gly Thr Asp Ala Lys Val Lys Leu Ile Lys
65                  70                  75                  80
Gln Glu Leu Asp Lys Tyr Lys Asn Ala Val Thr Glu Leu Gln Leu Leu
                85                  90                  95
Met Gln Ser Thr Pro Ala Thr Asn Asn Arg Ala Arg Arg Glu Leu Pro
            100                 105                 110
Arg Phe Met Asn Tyr Thr Leu Asn Asn Ala Lys Lys Thr Asn Val Thr
        115                 120                 125
Leu Ser Lys Lys Arg Lys Arg Arg Phe Leu Gly Phe Leu Leu Gly Val
    130                 135                 140
Gly Ser Ala Ile Ala Ser Gly Val Ala Val Ser Lys Val Leu His Leu
145                 150                 155                 160
Glu Gly Glu Val Asn Lys Ile Lys Ser Ala Leu Leu Ser Thr Asn Lys
                165                 170                 175
Ala Val Val Ser Leu Ser Asn Gly Val Ser Val Leu Thr Ser Lys Val
            180                 185                 190
Leu Asp Leu Lys Asn Tyr Ile Asp Lys Gln Leu Leu Pro Ile Val Asn
        195                 200                 205
Lys Gln Ser Cys Ser Ile Ser Asn Ile Glu Thr Val Ile Glu Phe Gln
    210                 215                 220
Gln Lys Asn Asn Arg Leu Leu Glu Ile Thr Arg Glu Phe Ser Val Asn
225                 230                 235                 240
Ala Gly Val Thr Thr Pro Val Ser Thr Tyr Met Leu Thr Asn Ser Glu
                245                 250                 255
Leu Leu Ser Leu Ile Asn Asp Met Pro Ile Thr Asn Asp Gln Lys Lys
            260                 265                 270
Leu Met Ser Asn Asn Val Gln Ile Val Arg Gln Gln Ser Tyr Ser Ile
        275                 280                 285
Met Ser Ile Ile Lys Glu Glu Val Leu Ala Tyr Val Val Gln Leu Pro
    290                 295                 300
Leu Tyr Gly Val Ile Asp Thr Pro Cys Trp Lys Leu His Thr Ser Pro
305                 310                 315                 320
Leu Cys Thr Thr Asn Thr Lys Glu Gly Ser Asn Ile Cys Leu Thr Arg
                325                 330                 335
Thr Asp Arg Gly Trp Tyr Cys Asp Asn Ala Gly Ser Val Ser Phe Phe
            340                 345                 350
Pro Gln Ala Glu Thr Cys Lys Val Gln Ser Asn Arg Val Phe Cys Asp
        355                 360                 365
Thr Met Asn Ser Leu Thr Leu Pro Ser Glu Val Asn Leu Cys Asn Val
    370                 375                 380
Asp Ile Phe Asn Pro Lys Tyr Asp Cys Lys Ile Met Thr Ser Lys Thr
385                 390                 395                 400
Asp Val Ser Ser Ser Val Ile Thr Ser Leu Gly Ala Ile Val Ser Cys
                405                 410                 415
Tyr Gly Lys Thr Lys Cys Thr Ala Ser Asn Lys Asn Arg Gly Ile Ile
            420                 425                 430
Lys Thr Phe Ser Asn Gly Cys Asp Tyr Val Ser Asn Lys Gly Val Asp
        435                 440                 445
Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480
Leu Val Phe Pro Ser Asp Glu Phe Asp Ala Ser Ile Ser Gln Val Asn
                485                 490                 495
Glu Lys Ile Asn Gln Ser Leu Ala Phe Ile Arg Lys Ser Asp Glu Leu
            500                 505                 510
Leu His Asn Val Asn Ala Gly Lys Ser Thr Thr Asn Arg Lys Arg Arg
        515                 520                 525
Ala Pro Val Lys Gln Thr Leu Asn Phe Asp Leu Leu Lys Leu Ala Gly
    530                 535                 540
Asp Val Glu Ser Asn Pro Gly Pro Met Ala Leu Ser Lys Val Lys Leu
545                 550                 555                 560
Asn Asp Thr Leu Asn Lys Asp Gln Leu Leu Ser Ser Ser Lys Tyr Thr
                565                 570                 575
Ile Gln Arg Ser Thr Gly Asp Ser Ile Asp Thr Pro Asn Tyr Asp Val
            580                 585                 590
Gln Lys His Ile Asn Lys Leu Cys Gly Met Leu Leu Ile Thr Glu Asp
        595                 600                 605
Ala Asn His Lys Phe Thr Gly Leu Ile Gly Met Leu Tyr Ala Met Ser
    610                 615                 620
Arg Leu Gly Arg Glu Asp Thr Ile Lys Ile Leu Arg Asp Ala Gly Tyr
625                 630                 635                 640
His Val Lys Ala Asn Gly Val Asp Val Thr Thr His Arg Gln Asp Ile
                645                 650                 655
Asn Gly Lys Glu Met Lys Phe Glu Val Leu Thr Leu Ala Ser Leu Thr
            660                 665                 670
Thr Glu Ile Gln Ile Asn Ile Glu Ile Glu Ser Arg Lys Ser Tyr Lys
        675                 680                 685
Lys Met Leu Lys Glu Met Gly Glu Val Ala Pro Glu Tyr Arg His Asp
    690                 695                 700
Ser Pro Asp Cys Gly Met Ile Ile Leu Cys Ile Ala Ala Leu Val Ile
705                 710                 715                 720
Thr Lys Leu Ala Ala Gly Asp Arg Ser Gly Leu Thr Ala Val Ile Arg
                725                 730                 735
Arg Ala Asn Asn Val Leu Lys Asn Glu Met Lys Arg Tyr Lys Gly Leu
            740                 745                 750
Leu Pro Lys Asp Ile Ala Asn Ser Phe Tyr Glu Val Phe Glu Lys Tyr
        755                 760                 765
Pro His Phe Ile Asp Val Phe Val His Phe Gly Ile Ala Gln Ser Ser
    770                 775                 780
Thr Arg Gly Gly Ser Arg Val Glu Gly Ile Phe Ala Gly Leu Phe Met
785                 790                 795                 800
Asn Ala Tyr Gly Ala Gly Gln Val Met Leu Arg Trp Gly Val Leu Ala
                805                 810                 815
Lys Ser Val Lys Asn Ile Met Leu Gly His Ala Ser Val Gln Ala Glu
            820                 825                 830
Met Glu Gln Val Val Glu Val Tyr Glu Tyr Ala Gln Lys Leu Gly Gly
        835                 840                 845
Glu Ala Gly Phe Tyr His Ile Leu Asn Asn Pro Lys Ala Ser Leu Leu
    850                 855                 860
Ser Leu Thr Gln Phe Pro His Phe Ser Ser Val Val Leu Gly Asn Ala
865                 870                 875                 880
Ala Gly Leu Gly Ile Met Gly Glu Tyr Arg Gly Thr Pro Arg Asn Gln
                885                 890                 895
Asp Leu Tyr Asp Ala Ala Lys Ala Tyr Ala Glu Gln Leu Lys Glu Asn
            900                 905                 910
Gly Val Ile Asn Tyr Ser Val Leu Asp Leu Thr Ala Glu Glu Leu Glu
        915                 920                 925
Ala Ile Lys His Gln Leu Asn Pro Lys Asp Asn Asp Val Glu Leu Gly
    930                 935                 940
Gly Gly Gly Ser Gly Gly Gly Gly Met Ser Arg Arg Asn Pro Cys Lys
945                 950                 955                 960
Phe Glu Ile Arg Gly His Cys Leu Asn Gly Lys Arg Cys His Phe Ser

965                 970                 975
His Asn Tyr Phe Glu Trp Pro Pro His Ala Leu Leu Val Arg Gln Asn
            980                 985                 990
Phe Met Leu Asn Arg Ile Leu Lys Ser Met Asp Lys Ser Ile Asp Thr
        995                 1000                1005
Leu Ser Glu Ile Ser Gly Ala Ala Glu Leu Asp Arg Thr Glu Glu Tyr
    1010                1015                1020
Ala Leu Gly Val Val Gly Val Leu Glu Ser Tyr Ile Gly Ser Ile Asn
1025                1030                1035                1040
Asn Ile Thr Lys Gln Ser Ala Cys Val Ala Met Ser Lys Leu Leu Thr
                1045                1050                1055
Glu Leu Asn Ser Asp Asp Ile Lys Lys Leu Arg Asp Asn Glu Glu Leu
            1060                1065                1070
Asn Ser Pro Lys Ile Arg Val Tyr Asn Thr Val Ile Ser Tyr Ile Glu
        1075                1080                1085
Ser Asn Arg Lys Asn Asn Lys Gln Thr Ile His Leu Leu Lys Arg Leu
    1090                1095                1100
Pro Ala Asp Val Leu Lys Lys Thr Ile Lys Asn Thr Leu Asp Ile His
1105                1110                1115                1120
Lys Ser Ile Thr Ile Asn Asn Pro Lys Glu Ser Thr Val Ser Asp Thr
                1125                1130                1135
Asn Asp His Ala Lys Asn Asn Asp Thr Thr
            1140                1145